(19) Europäisches Patentamt
European Patent Office
Office européen des brevets

(11) EP 4 726 049 A1

(12) # EUROPEAN PATENT APPLICATION
published in accordance with Art. 153(4) EPC

(43) Date of publication:
**15.04.2026 Bulletin 2026/16**

(21) Application number: **24819407.8**

(22) Date of filing: **07.06.2024**

(51) International Patent Classification (IPC):
***C12P 19/04*** *(2006.01)* ***A23C 9/123*** *(2006.01)*
***A23L 29/269*** *(2016.01)* ***C12N 1/20*** *(2026.01)*

(52) Cooperative Patent Classification (CPC):
**A23C 9/123; A23L 29/269; C12N 1/20; C12P 19/04**

(86) International application number:
**PCT/JP2024/020804**

(87) International publication number:
**WO 2024/253180 (12.12.2024 Gazette 2024/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **09.06.2023 JP 2023095345**

(71) Applicant: **MEIJI CO., LTD**
**Chuo-ku**
**Tokyo 104-8306 (JP)**

(72) Inventors:
- **YAMAMOTO Eri**
**Hachioji-shi, Tokyo 192-0919 (JP)**
- **TSUCHIYA Asami**
**Hachioji-shi, Tokyo 192-0919 (JP)**

(74) Representative: **TBK**
**Bavariaring 4-6**
**80336 München (DE)**

(54) **METHOD FOR PRODUCING EXOPOLYSACCHARIDE, AND USE OF SAME**

(57) Provided is a means for increasing a production amount of exopolysaccharides that can be applied to foods and is simpler. Provided is a method for producing an exopolysaccharide, the method including : treating an exopolysaccharide-producing bacterium under a condition that a lag phase is prolonged by 10% or more; and producing an exopolysaccharide by culturing the treated exopolysaccharide-producing bacterium in a medium. Provided is a composition containing any lactic acid bacterium of a bacterium belonging to *Lactobacillus delbrueckii* having an ability to produce an exopolysaccharide of 30 mg/kg or more, a bacterium belonging to *Streptococcus thermophilus* having an ability to produce an exopolysaccharide of 69 mg/kg or more, and a bacterium belonging to *Bifidobacterium breve* having an ability to produce an exopolysaccharide of 4.2 mg/kg or more.

*FIG. 1*

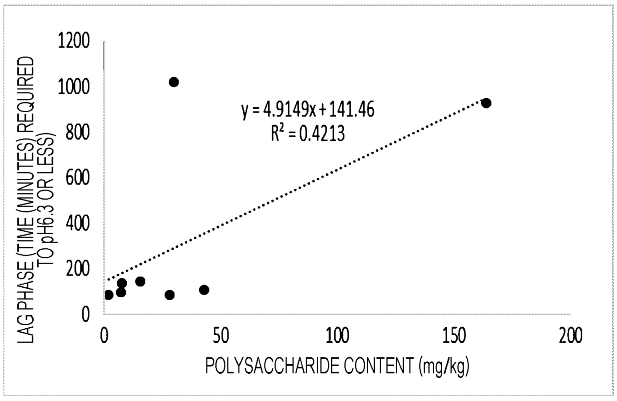


EP 4 726 049 A1

Processed by Luminess, 75001 PARIS (FR)

## Description

### TECHNICAL FIELD

The present invention relates to a method for producing an exopolysaccharide and use of the same.

### BACKGROUND ART

Exopolysaccharides (EPSs) produced by lactic acid bacteria are known to have various functionalities such as an immunomodulatory action, an action as a prebiotic, an anticancer action, and an action of decreasing cholesterol levels (Non Patent Document 1). Among these exopolysaccharides, polysaccharides produced by *a Lactobacillus delbrueckii* subsp. *bulgaricus* OLL1073R-1 strain (OLL1073R-1) are recognized to have an NK cell-activating action (Patent Document 1) and an anti-influenza effect (Patent Document 2). Therefore, it has been studied to increase a production amount of polysaccharides by lactic acid bacteria including OLL1073R-1. For example, there have been developed a medium for *Lactobacillus delbrueckii* subsp. *bulgaricus,* which is an exopolysaccharide-producing lactic acid bacterium, characterized in that formic acid and/or a formate is added to a skimmed milk and/or a reduced skimmed milk, and/or a skimmed milk and/or a skimmed milk powder is heat-treated, so that a total concentration of the formic acid and/or the formate is 0.4 to 10 mM (Patent Document 3); a method for increasing production of a lactic acid bacterium extracellular functional product, wherein a pH buffering agent that is an alkali metal salt of phosphoric acid in an amount within a range of 0.3 wt% or more and 0.55 wt% or less, and a mixture of a lactic acid bacterium of *Streptococcus thermophilus* species and a lactic acid bacterium of *Lactobacillus delbrueckii* subsp. *bulgaricus* species that produces a lactic acid bacterium extracellular functional product are blended with a milk raw material, and the milk raw material is fermented while suppressing growth of the lactic acid bacterium of *Streptococcus thermophilus* species by the pH buffering agent and promoting growth of the lactic acid bacterium of *Lactobacillus delbrueckii* subsp. *bulgaricus* species to increase production of the lactic acid bacterium extracellular functional product (Patent Document 4); and a method for increasing production of a lactic acid bacterium extracellular functional product, wherein a pH buffering agent that is an alkali metal salt of phosphoric acid in an amount within a range of 0.3 wt% or more and 0.55 wt% or less, and a mixture of a lactic acid bacterium of *Streptococcus thermophilus* species and a lactic acid bacterium of *Lactobacillus delbrueckii* subsp. *bulgaricus* species that produces a lactic acid bacterium extracellular functional product are blended with a milk raw material, and the milk raw material is fermented while suppressing growth of the lactic acid bacterium of *Streptococcus thermophilus* species by the pH buffering agent and promoting growth of the lactic acid bacterium of *Lactobacillus delbrueckii* subsp. *bulgaricus* species to increase production of the lactic acid bacterium extracellular functional product, and a fermented milk wherein an amount of EPS is 1.05 times or more and 4.2 times or less an amount of EPS contained in a fermented milk obtained by fermenting the raw material milk without degrading lactose contained in the raw material milk (Patent Document 5).

In addition, there have been studied a method for promoting a production amount of exopolysaccharides in a microorganism, the method including subjecting an exopolysaccharide-producing microorganism of the genus *Lactococcus* to a pulsed electric field treatment, wherein a condition for the pulsed electric field treatment includes an integrated value of a pulse width per 1 l of a culture solution and a total number of pulses (Patent Document 6); a method for producing an exopolysaccharide, characterized in that a lactic acid bacterium having an ability to produce an exopolysaccharide is inoculated into a medium obtained by saccharifying rice bran obtained when brown rice is milled at a percentage by weight of milled rice of 80% or more with malted rice or a saccharifying enzyme, and the lactic acid bacterium is fermented to produce an exopolysaccharide (Patent Document 7); a method for controlling a production amount of a neutral polysaccharide, which is one of exopolysaccharides (EPSs) of a lactic acid bacterium, characterized by including a step of culturing a lactic acid bacterium in a composition for lactic acid fermentation containing vitamin B2 (Patent Document 8); and the like.

Meanwhile, EPS is a biopolymer secreted by microorganisms including lactic acid bacteria in order to cope with severe environmental conditions, and is one of main components involved in formation of an extracellular biofilm matrix for protecting microorganisms from adverse conditions, and a relationship between production of EPS by lactic acid bacteria and environmental stress is known (Non Patent Document 2). It has been reported that exposure of stress-sensitive *Bifidobacterium bifidum* at a sublethal temperature of 42°C for 100 to 300 seconds significantly increases resistance of cells to lyophilization as compared to cells cultured in conventional bioreactors (Non Patent Document 3).

On the other hand, a lag phase is a period during which logarithmic growth is temporarily not observed in bacteria introduced into a new medium, and has been considered to be a preparatory stage for bacteria to take in nutrients and adapt to a new environment. Recent researches have found that the lag phase is a dynamic, systematic, adaptive, evolvable process that protects bacteria from threats and promotes their growth ability, and is widely related to researches on bacterial evolution, host-pathogen interactions, antibiotic resistance, environmental biology, molecular microbiology, and food safety (Non Patent Document 4). In addition, an influence of temperature on a growth rate of *Lactobacillus*

*rhamnosus GG* has been reported, and it has been reported that at 6 to 41°C, the lag phase of *L. rhamnosus GG* in milk has increased with decreasing temperature (Non Patent Document 5).

Incidentally, regarding the lag phase, a technique has been proposed in which, in production of a fermented milk, fermentation is performed in combination of adjusting a state of a fermented milk mix to which a fermented milk starter is added so that the lag phase of fermentation is longer than that of normal fermentation and reducing a dissolved oxygen concentration in the mix to 5 ppm or less after the addition of the starter, whereby fermentation is terminated when target acidity of lactic acid is reached in a normal fermentation time or a time shorter than the normal fermentation time without actually increasing the lag phase of fermentation, and as a result, a fermented milk having a smooth texture and a mild flavor with suppressed sourness and a rich body like cream cheese and having a hard tissue that does not collapse at a distribution stage is obtained (Patent Document 9).

PRIOR ART REFERENCES

Patent Documents

Patent Document 1: JP 2005-194259 A (JP 5177728 B2)
Patent Document 2: WO 2011/065300 A (JP 5971949 B2)
Patent Document 3: JP 2014-27925 A (JP 6209371 B2)
Patent Document 4: WO 2014-084340 A (JP 6392668 B2)
Patent Document 5: JP 2019-62782 A (JP 7109895 B2, JP 2022-103317 A)
Patent Document 6: JP 2017-2169276 A (JP 6621065 B2)
Patent Document 7: JP 2020-156341 A
Patent Document 8: JP 2022-45812 A
Patent Document 9: JP 2010-104376 A (JP 4759643 B2)


Non Patent Documents

Non Patent Document 1: Exopolysaccharides Produced by Lactic Acid Bacteria:From Biosynthesis to Health-Promoting Properties. Juraskova et al. Foods. 2022, 11, 156.
Non Patent Document 2: Exopolysaccharide production by lactic acid bacteria:the manipulation of environmental stresses for industrial applications. Phu-Tho Nguyen et al. AIMS Microbiology, 2020, 6(4):451-469.
Non Patent Document 3: Stochastic exposure to sub-lethal high temperature enhances exopolysaccharides (EPS) excretion and improves Bifidobacterium bifidum cell survival to freeze-drying. Huu Thanh Nguyen et al. Biochemical Engineering Journal, 2014,88:85-94.
Non Patent Document 4: Lag Phase Is a Dynamic, Organized, Adaptive, and Evolvable Period That Prepares Bacteria for Cell Division. Robert L. Bertrand. Journal of Bacteriology 2019, 201(7):e00697-18
Non Patent Document 5: Characterization of the growth of Lactobacillus rhamnosus GG in milk at suboptimal temperatures. L. M. Valik et al. Journal of Food and Nutrition Research, 2008 47(2):60-67


SUMMARY OF THE INVENTION

Object to be Achieved by the Invention

Some conventional methods for increasing a production amount of exopolysaccharides are to add special components to a culture system, and may be difficult to apply to foods. It is desirable to have a means for increasing a production amount of exopolysaccharides that can be applied to foods and is simpler.

Means for Achieving the Object

The present invention provides:

[1] A method for producing an exopolysaccharide, the method including a step of:

treating an exopolysaccharide-producing bacterium under a condition that a lag phase is prolonged by 10% or

more; and
producing an exopolysaccharide by culturing the treated exopolysaccharide-producing bacterium in a medium.

[2] The production method according to 1, wherein the step of producing an exopolysaccharide is performed for 4 to 72 hours.
[3] The production method according to 1 or 2, wherein the step of producing an exopolysaccharide includes a lag phase prolonged by 10%.
[4] The production method according to any one of 1 to 3, including at least one of a step of concentrating an exopolysaccharide and a step of purifying an exopolysaccharide.
[5] The production method according to any one of 1 to 4, wherein the exopolysaccharide-producing bacterium is any bacterium selected from a bacterium belonging to the genus *Lactobacillus,* a bacterium belonging to the genus *Lactococcus,* a bacterium belonging to the genus *Streptococcus,* and a bacterium belonging to the genus *Bifidobacterium.*
[6] The production method according to any one of 1 to 5, wherein the exopolysaccharide-producing bacterium is any bacterium selected from a bacterium belonging to *Lactobacillus delbrueckii,* a bacterium belonging to *Lactococcus lactis, a* bacterium belonging to *Streptococcus thermophilus,* and a bacterium belonging to *Bifidobacterium breve.*
[7] The production method according to any one of 1 to 6, wherein the *Lactobacillus delbrueckii* is a bacterium belonging to *Lactobacillus delbrueckii* ssp. *bulgaricus.*
[8] A composition containing an exopolysaccharide-producing bacterium of any of a bacterium belonging to the genus *Lactobacillus,* a bacterium belonging to the genus *Lactococcus,* a bacterium belonging to the genus *Streptococcus,* and a bacterium belonging to the genus *Bifidobacterium,* treated under a condition that a lag phase is prolonged by 10% or more, for use of an exopolysaccharide as a functional ingredient.
[9] The composition according to 8, wherein the exopolysaccharide-producing bacterium is any of a bacterium belonging to *Lactobacillus delbrueckii,* a bacterium belonging to *Lactococcus lactis,* a bacterium belonging to *Streptococcus thermophilus,* and a bacterium belonging to *Bifidobacterium breve.*
[10] A bacterium belonging to *Lactobacillus delbrueckii* having a lag phase of 152 minutes or more, a bacterium belonging to *Lactococcus lactis* having a lag phase of 236 minutes or more, a bacterium belonging to *Streptococcus thermophilus* having a lag phase of 199 minutes or more, and a bacterium belonging to *Bifidobacterium breve* having a lag phase of 425 minutes or more.
[11] A composition containing any of a bacterium belonging to *Lactobacillus delbrueckii* having an ability to produce an exopolysaccharide of 30 mg/kg or more, a bacterium belonging to *Lactococcus lactis* having an ability to produce an exopolysaccharide of 2.2 mg/kg or more, a bacterium belonging to *Streptococcus thermophilus* having an ability to produce an exopolysaccharide of 69 mg/kg or more, and a bacterium belonging to *Bifidobacterium breve* having an ability to produce an exopolysaccharide of 4.2 mg/kg or more.
[12] A culture of *Lactobacillus delbrueckii,* the culture containing 300 mg/kg or more of an exopolysaccharide produced by *Lactobacillus delbrueckii,* and a composition containing the culture.
[13] A culture of a bacterium belonging to *Lactococcus lactis,* the culture containing 2.2 mg/kg or more of an exopolysaccharide produced by *Lactococcus lactis,* and a composition containing the culture.
[14] A culture of *Streptococcus thermophilus,* the culture containing 69 mg/kg or more of an exopolysaccharide produced by *Streptococcus thermophilus,* and a composition containing the culture.
[15] A culture of *Bifidobacterium breve,* the culture containing 4.2 mg/kg or more of an exopolysaccharide produced by *Bifidobacterium breve,* and a composition containing the culture.
[16] A method for producing a fermented milk, the method including a step of fermenting a raw material milk with one or two or more lactic acid-producing bacteria including exopolysaccharide-producing bacteria treated so that a lag phase is prolonged by 10% or more to obtain a fermented milk containing an exopolysaccharide.
[17] The production method according to 16, wherein the step of producing an exopolysaccharide is performed for 4 to 72 hours.
[18] The production method according to 16 or 17, wherein the step of producing an exopolysaccharide includes a lag phase prolonged by 10%.
[19] The production method according to any one of 16 to 18, wherein the exopolysaccharide-producing bacteria treated so that a lag phase is prolonged by 10% or more include a bacterium belonging to *L. delbrueckii* ssp. *bulgaricus,* and a content of the exopolysaccharide in the fermented milk after the fermentation step is 30 mg/kg or more;

the exopolysaccharide-producing bacteria treated so that a lag phase is prolonged by 10% or more include a bacterium belonging to *Lactococcus lactis,* and a content of the exopolysaccharide in the fermented milk after the fermentation step is 2.2 mg/kg or more;
the exopolysaccharide-producing bacteria treated so that a lag phase is prolonged by 10% or more include a

bacterium belonging to *Streptococcus thermophilus,* and a content of the exopolysaccharide in the fermented milk after the fermentation step is 69 mg/kg or more; or

the exopolysaccharide-producing bacteria treated so that a lag phase is prolonged by 10% or more include a bacterium belonging to *Bifidobacterium breve,* and a content of the exopolysaccharide in the fermented milk after the fermentation step is 4.2 mg/kg or more.

[20] The production method according to any one of 16 to 19, wherein the lactic acid-producing bacteria further include a bacterium belonging to *Streptococcus thermophilus.*

[21] The production method according to any one of 16 to 20, wherein the lactic acid-producing bacteria further include a bacterium belonging to *L. bulgaricus.*

[22] A method for improving an ability to produce an exopolysaccharide of an exopolysaccharide-producing bacterium, the method including treating the exopolysaccharide-producing bacterium under a condition that a lag phase is prolonged.

[23] The production method according to any one of 1 to 7 and 16 to 21, or the method according to 22, wherein the treatment under a condition that a lag phase is prolonged includes any treatment selected from the group consisting of heat treatment, osmotic pressure treatment, and pH treatment.

The present invention also provides:

[1] A method for producing an exopolysaccharide, the method including a step of:

treating an exopolysaccharide-producing bacterium under a condition that a lag phase is prolonged by 10% or more; and

producing an exopolysaccharide by culturing the treated exopolysaccharide-producing bacterium in a medium.

[2] The production method according to 1, including at least one of a step of concentrating an exopolysaccharide and a step of purifying an exopolysaccharide.

[3] The production method according to 1, wherein the exopolysaccharide-producing bacterium is any bacterium selected from a bacterium belonging to the genus *Lactobacillus,* a bacterium belonging to the genus *Lactococcus,* a bacterium belonging to the genus *Streptococcus,* and a bacterium belonging to the genus *Bifidobacterium.*

[4] The production method according to 1, wherein the exopolysaccharide-producing bacterium is any bacterium selected from a bacterium belonging to *Lactobacillus delbrueckii,* a bacterium belonging to *Lactococcus lactis,* a bacterium belonging to *Streptococcus thermophilus,* and a bacterium belonging to *Bifidobacterium breve.*

[5] The production method according to 1, wherein the *Lactobacillus delbrueckii* is a bacterium belonging to *Lactobacillus delbrueckii* ssp. *bulgaricus.*

[6] A composition containing an exopolysaccharide-producing bacterium of any of a bacterium belonging to the genus *Lactobacillus,* a bacterium belonging to the genus *Lactococcus,* a bacterium belonging to the genus *Streptococcus,* and a bacterium belonging to the genus *Bifidobacterium,* treated under a condition that a lag phase is prolonged by 10% or more, for use of an exopolysaccharide as a functional ingredient.

[7] The composition according to 6, wherein the exopolysaccharide-producing bacterium is any of a bacterium belonging to *Lactobacillus delbrueckii,* a bacterium belonging to *Lactococcus lactis,* a bacterium belonging to *Streptococcus thermophilus,* and a bacterium belonging to *Bifidobacterium breve.*

[8] A bacterium belonging to *Lactobacillus delbrueckii* having a lag phase of 152 minutes or more, a bacterium belonging to *Lactococcus lactis* having a lag phase of 236 minutes or more, a bacterium belonging to *Streptococcus thermophilus* having a lag phase of 199 minutes or more, and a bacterium belonging to *Bifidobacterium breve* having a lag phase of 425 minutes or more.

[9] A composition containing any of a bacterium belonging to *Lactobacillus delbrueckii* having an ability to produce an exopolysaccharide of 30 mg/kg or more, a bacterium belonging to *Lactococcus lactis* having an ability to produce an exopolysaccharide of 2.2 mg/kg or more, a bacterium belonging to *Streptococcus thermophilus* having an ability to produce an exopolysaccharide of 69 mg/kg or more, and a bacterium belonging to *Bifidobacterium breve* having an ability to produce an exopolysaccharide of 4.2 mg/kg or more.

[10] A culture of *Lactobacillus delbrueckii,* the culture containing 300 mg/kg or more of an exopolysaccharide, and a composition containing the culture. Preferably, a culture of *Lactobacillus delbrueckii,* the culture containing 300 mg/kg or more of an exopolysaccharide produced by *Lactobacillus delbrueckii,* and a composition containing the culture.

[11] A culture of a bacterium belonging to *Lactococcus lactis,* the culture containing 2.2 mg/kg or more of an exopolysaccharide, and a composition containing the culture. Preferably, a culture of a bacterium belonging to *Lactococcus lactis,* the culture containing 2.2 mg/kg or more of an exopolysaccharide produced by a bacterium belonging to *Lactococcus lactis,* and a composition containing the culture.

[12] A culture of *Streptococcus thermophilus,* the culture containing 69 mg/kg or more of an exopolysaccharide, and a composition containing the culture. Preferably, a culture of *Streptococcus thermophilus,* the culture containing 69 mg/kg or more of an exopolysaccharide produced by *Streptococcus thermophilus,* and a composition containing the culture.

[13] A culture of *Bifidobacterium breve,* the culture containing 4.2 mg/kg or more of an exopolysaccharide, and a composition containing the culture. Preferably, a culture of *Bifidobacterium breve,* the culture containing 4.2 mg/kg or more of an exopolysaccharide produced by *Bifidobacterium breve,* and a composition containing the culture.

[14] A method for producing a fermented milk, the method including a step of fermenting a raw material milk with one or two or more lactic acid-producing bacteria including exopolysaccharide-producing bacteria treated so that a lag phase is prolonged by 10% or more to obtain a fermented milk containing an exopolysaccharide.

[15] The production method according to 14, wherein the exopolysaccharide-producing bacteria treated so that a lag phase is prolonged by 10% or more include a bacterium belonging to *L. delbrueckii* ssp. *bulgaricus,* and a content of the exopolysaccharide in the fermented milk after the fermentation step is 30 mg/kg or more;

the exopolysaccharide-producing bacteria treated so that a lag phase is prolonged by 10% or more include a bacterium belonging to *Lactococcus lactis,* and a content of the exopolysaccharide in the fermented milk after the fermentation step is 2.2 mg/kg or more;

the exopolysaccharide-producing bacteria treated so that a lag phase is prolonged by 10% or more include a bacterium belonging to *Streptococcus thermophilus,* and a content of the exopolysaccharide in the fermented milk after the fermentation step is 69 mg/kg or more; or

the exopolysaccharide-producing bacteria treated so that a lag phase is prolonged by 10% or more include a bacterium belonging to *Bifidobacterium breve,* and a content of the exopolysaccharide in the fermented milk after the fermentation step is 4.2 mg/kg or more.

[16] The production method according to 14 or 15, wherein the lactic acid-producing bacteria further include a bacterium belonging to *Streptococcus thermophilus.*

[17] The method according to any one of 14 to 16, wherein the lactic acid-producing bacteria further include a bacterium belonging to *L. bulgaricus.*

[18] A method for improving an ability to produce an exopolysaccharide of an exopolysaccharide-producing bacterium, the method including treating the exopolysaccharide-producing bacterium under a condition that a lag phase is prolonged.

[19] The method according to 18, wherein the treatment under a condition that a lag phase is prolonged includes any treatment selected from the group consisting of heat treatment, osmotic pressure treatment, and pH treatment.

## Effects of the Invention

It is possible to efficiently produce an exopolysaccharide of an exopolysaccharide-producing bacterium.

By a simple treatment, an ability to produce an exopolysaccharide of an exopolysaccharide-producing bacterium can be enhanced.

By increasing an amount of an exopolysaccharide, an intended function in foods can be improved.

## BRIEF DESCRIPTION OF THE DRAWINGS

Fig. 1 shows an increase in polysaccharide content in a fermented product of a lactic acid bacterium (pretreatment with high temperature, use of skimmed milk powder medium).

Fig. 2 shows an increase in polysaccharide content in a fermented product of a lactic acid bacterium (pretreatment with high temperature, use of synthetic medium).

Fig. 3 shows an increase in polysaccharide content in a fermented product of a lactic acid bacterium (pretreatment at 60°C for 3 to 10 minutes or 80°C for 1 to 3 minutes).

Fig. 4 shows an increase in polysaccharide content in a fermented product of a lactic acid bacterium (pretreatment with high osmotic pressure).

Fig. 5 shows an increase in polysaccharide content in a fermented product of a lactic acid bacterium (pretreatment with high osmotic pressure).

Fig. 6 shows an increase in polysaccharide content in a fermented product of a lactic acid bacterium (when culture is started in a medium adjusted to high pH).

Fig. 7 shows changes in pH during fermentation of a lactic acid bacterium (pretreatment with high temperature, use of

skimmed milk powder medium).

Fig. 8 shows a viable cell count in a fermented product of a lactic acid bacterium (pretreatment with high temperature, use of skimmed milk powder medium).

Fig. 9 shows an increase in polysaccharide content in a fermented product of a lactic acid bacterium (pretreatment with high temperature, use of skimmed milk powder medium).

## MODES FOR CARRYING OUT THE INVENTION

Hereinafter, the present invention will be described in detail based on an embodiment of the present invention (referred to as the present embodiment). The following embodiments are examples for describing the present invention, and are not intended to limit the present invention only to these embodiments. In the present invention, the term "any" means that type and number are arbitrary. Unless otherwise specified, % for content or concentration is based on mass.

### [Method for producing exopolysaccharide]

The present embodiment relates to a method for producing an exopolysaccharide, the method including: treating an exopolysaccharide-producing bacterium under a condition that a lag phase is prolonged; and producing an exopolysaccharide by culturing the treated exopolysaccharide-producing bacterium in a medium.

### <Applicable exopolysaccharide-producing bacterium>

The exopolysaccharide-producing bacterium is not particularly limited as long as it is a bacterium having an ability to produce an exopolysaccharide (EPS), and a lactic acid bacterium is preferable. Incidentally, in the present invention, a lactic acid bacterium among exopolysaccharide-producing bacteria may be described as an example, and the description also applies to exopolysaccharide-producing bacteria other than a lactic acid bacterium. The exopolysaccharide-producing bacterium used in the present embodiment is not particularly limited as long as it is a bacterium that is used in food production and has an ability to produce an exopolysaccharide (EPS). The exopolysaccharide-producing bacterium may be a bacillus (bacterium belonging to the genus *Lactobacillus,* or the like) or a coccus (bacterium belonging to the genus *Lactococcus, Leuconostoc, Bediococcus,* or *Streptococcus,* or the like). The exopolysaccharide-producing bacterium may also be a bacterium belonging to the genus *Bifidobacterium.* In the present embodiment, as the exopolysaccharide-producing bacterium, only one type may be used, or two or more types may be used.

In one embodiment, the exopolysaccharide-producing bacterium is any bacterium selected from a bacterium belonging to the genus *Lactobacillus,* a bacterium belonging to the genus *Lactococcus,* a bacterium belonging to the genus *Streptococcus,* a bacterium belonging to the genus *thermophilus,* and a bacterium belonging to the genus *Bifidobacterium.*

One example of a preferred exopolysaccharide-producing bacterium is a bacterium belonging to the genus *Lactobacillus.* Examples of the *Lactobacillus* bacterium include *bulgaricus* species, *casei* species, *acidophilus* species, and *plantarum* species. Incidentally, in the present specification, "bacterium belonging to the genus *Lactobacillus"* refers to a lactic acid bacterium belonging to any of the genera of 25 genera newly set by rearrangement of lactic acid bacteria published in the article "A taxonomic note on the genus Lactobacillus:Description of 23 novel genera, emended description of the genus Lactobacillus Beijerinck 1901, and union of Lactobacillaceae and Leuconostocaceae" in INTERNATIONAL JOURNAL OF SYSTEMATIC AND EVOLUTIONARY MICROBIOLOGY, Volume 70, Issue 4 issued on April 15, 2020, that is, the genus *Lactobacillus,* the genus *Paralactobacillus,* the genus *Holzapfelia,* the genus *Amylolactobacillus,* the genus *Bombilactobacillus,* the genus *Companilactobacillus,* the genus *Lapidilactobacillus,* the genus *Agrilactobacillus,* the genus *Schleiferilactobacillus,* the genus *Loigolactobacilus,* the genus *Lacticaseibacillus,* the genus *Latilactobacillus,* the genus *Dellaglioa,* the genus *Liquorilactobacillus,* the genus *Ligilactobacillus,* the genus *Lactiplantibacillus,* the genus *Furfurilactobacillus*, the genus *Paucilactobacillus,* the genus *Limosilactobacillus,* the genus *Fructilactobacillus,* the genus *Acetilactobacillus,* the genus *Apilactobacillus,* the genus *Levilactobacillus,* the genus *Secundilactobacillus,* and the genus *Lentilactobacillus.* In one embodiment, a bacterium obtained by excluding the genus *Lacticaseibacillus,* particularly *Lacticaseibacillus rhamnosus,* more specifically, *Lactobacillus rhamnosus GG* described in Non Patent Document 5 (after rearrangement according to the above article, it is classified as *Lacticaseibacillus rhamnosus*) from a bacterium belonging to the genus *Lactobacillus* may be used as an exopolysaccharide-producing bacterium.

Among these bacteria belonging to the genus *Lactobacillus,* a bacterium belonging to *Lactobacillus delbrueckii* is preferable, and a bacterium belonging to *Lactobacillus delbrueckii* subsp. *bulgaricus* is more preferable.

In a particularly preferred embodiment, the exopolysaccharide-producing bacterium is *Lactobacillus delbrueckii* subsp. *bulgaricus* OLL1073R-1 (accession No.: FERM BP-10741) (it may be referred to as OLL1073R-1).

OLL1073R-1 has been internationally deposited at International Patent Organism Depositary (IPOD), National Institute of Technology and Evaluation (NITE) (2-5-8 Kazusakamatari, Kisarazu-shi, Chiba, Japan) under the Budapest

Treaty (depositor: Meiji Co., Ltd., date of deposit: November 29, 2006, accession No.: FERM BP-10741).

Other preferred strains of the bacterium belonging to *Lactobacillus delbrueckii* or the bacterium belonging to *Lactobacillus delbrueckii* subsp. *bulgaricus* include the following.

Lactobacillus delbrueckii subsp. bulgaricus OLL205013 (NITE BP-02411),
Lactobacillus delbrueckii subsp. bulgaricus OLL1171 (NITE BP-01569),
*Lactobacillus delbrueckii* OLL204989 (NITE BP-02874)

One of the other examples of a preferred exopolysaccharide-producing bacterium is any bacterium selected from a bacterium belonging to *Lactococcus lactis,* a bacterium belonging to *Streptococcus thermophilus,* and a bacterium belonging to *Bifidobacterium breve.*

Examples of a preferred strain belonging to *Lactococcus lactis* include the following.
*Lactococcus lactis* spp. *lactis* OLS3789 (NITE BP-1387)

Examples of a preferred strain belonging to *Streptococcus thermophilus* include the following.

Streptococcus thermophilus OLS3618 (NITE BP-01815),
Streptococcus thermophilus OLS 3290 (FERM BP-19638)

Examples of a preferred strain belonging to *Bifidobacterium breve* include the following.

*B. breve* JCM1192$^T$
JCM1192$^T$ is a reference strain, and is available from Microbe Division, Riken BioResource Research Center (Japan Collection of Microorganisms, JCM).

<Step of pretreating exopolysaccharide-producing bacterium>

The method for producing EPS according to the present embodiment includes a step of pretreating an exopolysaccharide-producing bacterium under a condition that a lag phase is prolonged before culturing the exopolysaccharide-producing bacterium in a predetermined medium. The present inventors have found that, in culture of an exopolysaccharide-producing bacterium, pretreating an exopolysaccharide-producing bacterium before culture, specifically, subjecting the bacterium to a treatment under a condition that a lag phase in culture is prolonged, increases a concentration of EPS produced in a culture solution as compared with that in an untreated case, and that there is a positive correlation between a length of the lag phase and the concentration of EPS in the culture solution (see Figs. 1 to 6). Incidentally, "untreated" may be referred to as "without treatment".

EPS is a biopolymer secreted by microorganisms including lactic acid bacteria in order to cope with severe environmental conditions, and is one of main components involved in formation of an extracellular biofilm matrix for protecting microorganisms from adverse conditions, and a relationship between production of EPS by lactic acid bacteria and environmental stress is known (Non Patent Document 2, supra). It has been reported that exposure of stress-sensitive *Bifidobacterium bifidum* at a sublethal temperature of 42°C for 100 to 300 seconds significantly increases resistance of cells to lyophilization as compared to cells cultured in conventional bioreactors (Non Patent Document 3, supra).

The lag phase generally refers to a period during which logarithmic growth is temporarily not observed in bacteria introduced into a new medium. Recent researches have found that the lag phase is a dynamic, systematic, adaptive, evolvable process that protects bacteria from threats and promotes their growth ability, and is widely related to researches on bacterial evolution, host-pathogen interactions, antibiotic resistance, environmental biology, molecular microbiology, and food safety (Non Patent Document 4, supra).

In the production method of the present embodiment, regarding whether or not a certain pretreatment condition can be said to be a condition that a lag phase is prolonged, a condition in which a time (lag phase) required for pH of a culture solution to decrease by a predetermined degree in an initial stage of culture (for a while from a start of culture) becomes longer when a bacterium treated under the condition is cultured than when the bacterium is not treated can be said to be a condition that a lag phase is prolonged. More specifically, the time required for pH of a culture solution to decrease by a predetermined degree can be a time required for the pH of the culture solution at a start of culture to decrease by 0.2. The pH of the culture solution at the start of culture can be pH of a fresh medium. Incidentally, pH can vary depending on a temperature, and when a pH value in the present invention is indicated, the pH value is a value measured at a temperature at the time of culture of an exopolysaccharide-producing bacterium unless otherwise specified. This temperature is often 40°C, and is 30°C for *Lactococcus lactis.*

A delay rate in the lag phase can be defined as follows.

Delay rate in lag phase = [(Time (minutes) required for treated bacterium to change pH in initial stage of culture by 0.2) - (Time (minutes) required for untreated bacterium to change pH in initial stage of culture by 0.2)]/(Time (minutes) required for untreated bacterium to change pH in initial stage of culture by 0.2)

When the delay rate is determined, culture for determining the time required for a treated bacterium to change pH in an initial stage of culture by 0.2, and culture for determining the time required for an untreated bacterium to change pH in an initial stage of culture by 0.2 are performed under the same culture conditions using the same medium. The medium and the culture conditions can be those used as standard for an untreated bacterium or those used to produce EPS. Examples of a medium that can be used in determining the delay rate are an MRS medium for a bacterium belonging to *L. delbrueckii,* an MRS medium for a bacterium belonging to *L. lactis,* an M17 medium for a bacterium belonging to *S. thermophilus,* and a GAM medium for a bacterium belonging to *B. breve.* When the delay rate is determined, a skimmed milk powder medium may be used if appropriate.

In one embodiment, regardless of which exopolysaccharide-producing bacterium is used, and regardless of which medium is used in culture after treatment, in order to obtain an intended effect, it is preferable to perform treatment under a condition that a lag phase is prolonged by 10% or more, it is more preferable to perform treatment under a condition that a lag phase is prolonged by 30% or more, it is more preferable to perform treatment under a condition that a lag phase is prolonged by 50% or more, it is more preferable to perform treatment under a condition that a lag phase is prolonged by 70% or more, and it is still more preferable to perform treatment under a condition that a lag phase is prolonged by 90% or more.

The production method of the present embodiment can be achieved by treating an exopolysaccharide-producing bacterium under a condition that a lag phase of the exopolysaccharide-producing bacterium is prolonged. Treating an exopolysaccharide-producing bacterium under a condition that a lag phase of the exopolysaccharide-producing bacterium is prolonged (prolongation of the lag phase) includes high-temperature treatment, low-temperature treatment, high-osmotic pressure treatment, low-osmotic pressure treatment, low-pH treatment, high-pH treatment, highpressure treatment, drying treatment, freezing treatment, freeze-thawing treatment, nutrient deprivation treatment (starvation of nutrients such as nitrogen, sugar, and carbon dioxide), excessive nutrient supply, $CO_2$ treatment, oxidation load, co-culture treatment, chemical substance treatment, antibiotic treatment, cell wall-degrading enzyme treatment, antibacterial peptide treatment, ultrasonic treatment, and ultraviolet irradiation treatment of the exopolysaccharide-producing bacterium. As the condition that a lag phase of the exopolysaccharide-producing bacterium is prolonged, only one type may be used, or two or more types may be used.

Prolongation of a lag phase is performed by any treatment selected from heat (high-temperature) treatment, osmotic pressure (high-osmotic pressure) treatment, and pH (high-pH) treatment from the viewpoint that an intended effect is found to be relatively high and it is easy to perform.

(Heat)

In one embodiment, the condition that a lag phase is prolonged by 10% or more is heat (high-temperature) treatment regardless of which exopolysaccharide-producing bacterium is used and regardless of which medium is used in culture after treatment.

In a case of a bacterium other than a bacterium vulnerable to high temperature (the bacterium can endure treatment at 60°C for 10 minutes, that is, when the bacterium is cultured after treatment at 60°C for 10 minutes, a time required for pH of a culture solution to decrease by 0.2 as compared with that in an initial stage of culture is within 24 hours), a temperature is preferably 45°C or higher, more preferably 50°C or higher, and still more preferably 58°C or higher. An upper limit value of the temperature is preferably 86°C or lower, more preferably 84°C or lower, and still more preferably 82°C or lower.

In a case of a bacterium vulnerable to high temperature, the temperature is preferably 40°C or higher, more preferably 45°C or higher, and still more preferably 50°C or higher. The upper limit value of the temperature is preferably 76°C or lower, more preferably 74°C or lower, still more preferably 72°C or lower, and particularly preferably 70°C or lower.

More specifically, in a case of a bacterium belonging to *Lactobacillus delbrueckii, Streptococcus thermophilus,* or *Bifidobacterium breve,* the temperature is preferably 50 to 86°C, and more preferably 58 to 82°C. In a case of a bacterium belonging to *Lactococcus lactis,* the temperature is preferably 45 to 70°C, and more preferably 50 to 70°C.

When the heat treatment is performed, a treatment time can be appropriately determined according to the temperature. In a case of a bacterium other than a bacterium vulnerable to high temperature, when the bacterium is treated at 58 to 62°C, the treatment time is preferably 0.5 minutes or more, preferably 1 minute or more, preferably 2 minutes or more, preferably 3 minutes or more, more preferably 4 minutes or more, and still more preferably 8 minutes or more. An upper limit value is preferably 20 minutes or less, more preferably 18 minutes or less, preferably 16 minutes or less, and still more preferably 14 minutes or less. When the bacterium is treated at 78 to 82°C, the treatment time is preferably 0.5

minutes or more, preferably 1 minute or more, more preferably 2 minutes or more, and still more preferably 2.5 minutes or more. The upper limit value is preferably 6 minutes or less, more preferably 5 minutes or less, and still more preferably 4 minutes or less.

In a case of a bacterium vulnerable to high temperature, when the bacterium is treated at 50 to 70°C, the treatment time is preferably 0.25 seconds or more, and can be, for example, 0.5 minutes or more or 0.75 minutes or more. The upper limit value is preferably 6 minutes or less, and can be, for example, 5 minutes or less, 4 minutes or less, or 3 minutes or less.

More specifically, when a bacterium belonging to *Lactobacillus delbrueckii, Streptococcus thermophilus,* or *Bifidobacterium breve* is treated at 58 to 62°C, the treatment time is preferably 0.5 to 20 minutes, more preferably 2.5 to 18 minutes, and still more preferably 3 to 10 minutes. When a bacterium belonging to *Lactobacillus delbrueckii, Streptococcus thermophilus,* or *Bifidobacterium breve* is treated at 78 to 82°C, the treatment time is preferably 0.5 to 6 minutes, more preferably 0.5 to 16 minutes, and still more preferably 1 to 3 minutes. When a bacterium belonging to *Lactococcus lactis* is treated at 58 to 62°C, the treatment time is preferably 0.5 to 2 minutes, and more preferably 0.5 to 1 minute.

(Osmotic pressure)

In one embodiment, the condition that a lag phase is prolonged by 10% or more is high-osmotic pressure treatment (high-salt concentration treatment) regardless of which exopolysaccharide-producing bacterium is used and regardless of which medium is used in culture after treatment. A salt concentration is preferably 0.8% or more, more preferably 3% or more, and still more preferably 5% or more. An upper limit value is preferably 20% or less, more preferably 16% or less, and still more preferably 12% or less.

When the high-salt concentration treatment is performed, a treatment time can be appropriately determined according to the salt concentration. The treatment time is preferably 1 hour or more, more preferably 2 hours or more, and still more preferably 3 hours or more. An upper limit value is preferably 9 hours or less, more preferably 7 hours or less, and still more preferably 3 hours or less.

(pH)

In one embodiment, the condition that a lag phase is prolonged by 10% or more is high-pH treatment regardless of which exopolysaccharide-producing bacterium is used and regardless of which medium is used in culture after treatment. The high-pH treatment can be performed by setting pH at a start of culture to be high. The pH at the start of culture is preferably more than 6.3, more preferably 6.5 or more, and still more preferably 6.8 or more. An upper limit value is preferably 7.4 or less, more preferably 7.2 or less, and still more preferably 7.0 or less.

(Other conditions that lag phase is prolonged)

Examples of treatment other than heat, osmotic pressure, and pH that is considered to have a relatively high intended effect, are treatments with any one selected from the group consisting of ultrasonic waves, ultraviolet rays, high pressure, cell wall-degrading enzymes, antibacterial peptides, low temperature, and freeze-thawing. It is considered that the intended effect cannot be obtained by treatment of exposing an exopolysaccharide-producing bacterium to a low temperature, for example, 21°C or lower for a short time, for example, several minutes, but the intended effect can be expected by treatment of exposing an exopolysaccharide-producing bacterium to a low temperature for several hours, for example, 4 hours or more.

In one embodiment, the step of pretreating an exopolysaccharide-producing bacterium does not include refrigerated storage, freezing treatment, and lyophilization treatment, or is treatment other than these. In addition, in the method for producing an exopolysaccharide, although a lag phase can be prolonged by relatively reducing an amount of bacteria added to a medium at a start of culture and culturing at a relatively low temperature, reducing the amount of bacteria added to a medium and culturing at a relatively low temperature do not correspond to the step of pretreating an exopolysaccharide-producing bacterium.

<Step of producing exopolysaccharide>

The production method of the present embodiment includes a step of culturing an exopolysaccharide-producing bacterium treated under a condition that a lag phase is prolonged by 10% or more in a medium to produce an exopolysaccharide. The condition of this step can be the same as that in a case of culturing for producing EPS by a bacterium that is not treated.

As a medium for producing EPS, a medium containing a milk raw material may be used, or a synthetic medium may be used. In order to obtain EPS in a form of a fermented milk, a medium containing a milk raw material can be used. A

method for producing a fermented milk will be described later. When a synthetic medium is used, the synthetic medium can be selected according to an exopolysaccharide-producing bacterium to be used.

Culture may be in anaerobic or aerobic conditions. A dissolved oxygen concentration in a medium is not particularly limited as long as a lag phase prolonged by 10% or more is secured. Although depending on bacteria to be used, it is considered that growth of lactic acid bacteria is promoted and the lag phase is shortened when the dissolved oxygen concentration is low (Patent Document 9). Therefore, it is preferable that the dissolved oxygen concentration in a medium is not too low from the viewpoint of securing a prolonged lag phase and the like. In one embodiment, regardless of other culture conditions, the dissolved oxygen concentration in a medium at a start of culture can be 1 ppm or more, 2 ppm or more, 3 ppm or more, 4 ppm or more, 5 ppm or more, more than 5 ppm, 5.1 ppm or more, 5.2 ppm or more, 5.3 ppm or more, 5.4 ppm or more, or 5.5 ppm or more. In addition, the dissolved oxygen concentration in a medium at a start of culture can be, regardless of other culture conditions, 10 ppm or less, and may be 9 ppm or less, 8 ppm or less, 7 ppm or less, or 6 ppm or less. In addition, in another embodiment, regardless of other culture conditions, the method for producing an exopolysaccharide of the present embodiment does not include a treatment or a step of reducing dissolved oxygen in a medium.

A culture temperature is preferably 37 to 43°C, and a culture time is preferably 4 to 72 hours, and can be, for example, 4 to 48 hours, 4 to 36 hours, 4 to 24 hours, 6 to 72 hours, 6 to 48 hours, 6 to 36 hours, 6 to 24 hours, 8 to 72 hours, 8 to 48 hours, 8 to 36 hours, 8 to 24 hours, 12 to 72 hours, 12 to 48 hours, 12 to 36 hours, 12 to 24 hours, 14 to 72 hours, 14 to 48 hours, 14 to 36 hours, 14 to 24 hours, 16 to 36 hours, or 16 to 24 hours, from the viewpoint of growth of an exopolysaccharide-producing bacterium and EPS to be produced. When the culture time is 4 hours or more, sufficient EPS can be obtained. In addition, when the culture time is 48 hours or less, a decrease in viable cell count of an exopolysaccharide-producing bacterium is small, and an increase in production amount of EPS according to time is expected. pH of a culture solution is preferably 3.5 to 7.5, more preferably 4.5 to 7.0, and still more preferably pH 5.5 to 6.5 throughout a culture period. Since the pH of a culture solution of an exopolysaccharide-producing bacterium usually decreases with the culture time, it may be possible to further increase the production amount of EPS by maintaining the pH by performing neutralization during culture.

The step of producing an exopolysaccharide in the production method of the present embodiment can include a lag phase prolonged by 10% or more. The lag phase prolonged by 10% or more refers to a lag phase prolonged by 10% or more as compared with a case where the same bacteria that have not been pretreated are cultured under the same conditions. The lag phase referred to herein may be a period during which logarithmic growth is not temporarily observed in bacteria introduced into a new medium, or may be a time required for pH at a start of culture to decrease by a predetermined degree, for example, 0.2.

In the step of producing an exopolysaccharide, even in a case of using an exopolysaccharide-producing bacterium treated under a condition that a lag phase is prolonged by 10% or more, it is considered that when culture is performed under a condition that growth of bacteria is promoted and the lag phase is shortened, an actual length of the lag phase may be about the same as or shorter than that in a case of using untreated bacteria. In such a case, the step of producing an exopolysaccharide cannot be said to include a lag phase prolonged by 10% or more although treated bacteria are used. In one embodiment, the step of producing an exopolysaccharide preferably includes a lag phase prolonged by 10% or more, more preferably includes a lag phase prolonged by 30% or more, and still more preferably includes a lag phase prolonged by 90% or more, regardless of bacteria to be used and culture conditions.

Incidentally, the lag phase can be prolonged by relatively reducing an amount of lactic acid bacteria in a medium at a start of culture (Patent Document 9) and culturing at a relatively low temperature (Non Patent Document 5), but in one embodiment, the step of producing an exopolysaccharide in the production method of the present embodiment does not include a lag phase prolonged by reducing an amount of bacteria added to a medium at a start of culture (or includes a lag phase prolonged by means other than reducing an amount of bacteria in a medium at a start of culture) and does not include a lag phase prolonged by culturing at a relatively low temperature (or includes a lag phase prolonged by means other than culturing at a relatively low temperature) regardless of bacteria to be used and other culture conditions. In addition, production of an exopolysaccharide can be increased by reducing lactose in a raw material milk used for a medium (Patent Document 5), but in one embodiment, the step of producing an exopolysaccharide in the production method of the present embodiment does not include an embodiment of using a raw material milk in which lactose has been previously reduced (or production of an exopolysaccharide is increased by means other than using a raw material milk in which lactose has been previously reduced) regardless of bacteria to be used and other culture conditions.

<Other steps and the like>

The production method of the present embodiment may include steps other than those described above, and examples of such steps include a step of filtering a culture solution obtained by culturing a bacterium, a step of centrifugation, a step of membrane separation, a step of sterilization, a step of concentration, a step of purifying produced EPS, a step of drying, and the like.

The purification step may include:

1. Bacterial cells are removed from the culture by centrifugation.
2. Trichloroacetic acid is added so that the final concentration is about 5 to 10 wt%, protein precipitation is performed, and centrifugation is performed.
3. High-molecular weight polysaccharides and proteins are recovered as precipitates by ethanol precipitation.
4. Proteins and nucleic acids are removed.

    a) Nucleic acids are subjected to degradation treatment with DNase and RNase.
    b) Proteins are degraded with proteinase.
    c) After proteins are thermally denatured, centrifugation and dialysis are performed.

5. Acidic polysaccharides are adsorbed with an anion exchange resin, and then eluted and recovered.

In addition, for example, when only a neutral polysaccharide is used, a neutral polysaccharide isolated by the method described in JP 2000-247895 A or the like and purified as necessary can be used.
EPS can also be isolated by the following procedure.

1. Trichloroacetic acid is added to a medium at a final concentration of 10 wt% to denature proteins.
2. The denatured proteins and bacterial cells are removed from the culture by centrifugation.
3. A high-molecular weight polysaccharide is precipitated by ethanol precipitation and recovered.
4. Acidic polysaccharides are adsorbed by an anion exchange resin, and neutral polysaccharides are recovered from the remaining eluate.
5. Nucleic acids are degraded by DNase and RNase treatment.
6. Proteins are degraded by proteinase treatment.
7. The enzyme is deactivated by heating at 90°C for 10 minutes.
8. A neutral polysaccharide is purified by ethanol precipitation and dialysis.

<EPS>

EPS produced by an exopolysaccharide-producing bacterium in the production method of the present embodiment can be various. EPS produced by an exopolysaccharide-producing bacterium is structurally classified into those that are homopolysaccharides and those that are heteropolysaccharides (e.g., those composed of galactose and glucose), and may be subjected to modifications such as phosphorylation and sulfation, but any EPS can be produced by the production method of the present embodiment.
In one embodiment, EPS contains at least one of a neutral polysaccharide and an acidic polysaccharide in which a phosphate group is added to the neutral polysaccharide. Such EPS is known to be produced by *Lactobacillus delbrueckii* subsp. *bulgaricus, Lactococcus lactis* spp. *lactis, Lactococcus lactis* subsp. *cremoris,* and the like.
In one embodiment, EPS may contain an acidic exopolysaccharide having a repeating structure in which repeating units represented by the following formula (I) are linked.

[Chem. 1]

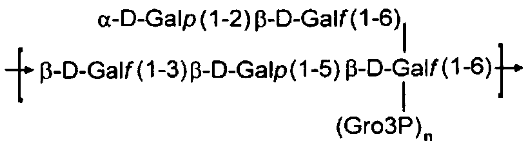

... (I)

In formula (I), n independently represents an integer of 0 or 1 for each repeating unit. That is, individual repeating units represented by formula (I) constituting acidic EPS have one glycerol 3-phosphate group or have no glycerol 3-phosphate group, but at least one glycerol 3-phosphate group is added to the entire acidic polysaccharide.
The acidic polysaccharide may have a repeating structure represented by the following formula (II) in which repeating units represented by formula (I) are linked.

[Chem. 2]

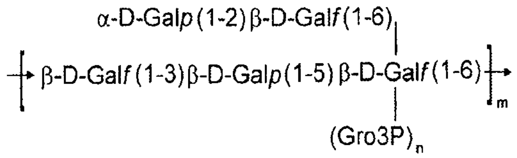

... (II)

In formula (II), n independently represents an integer of 0 or 1 for each repeating unit. In formula (II), m is an integer, and may be 1 to 300 or 1 to 200.

In formulae (I) and (II), α-D-Galp represents a pyranose-type α-D-galactose residue, β-D-Galp represents a pyranose-type β-D-galactose residue, β-D-Galf represents a furanose-type β-D-galactose residue, and Gro3P represents a glycerol 3-phosphate group. (1-2), (1-3), (1-5), and (1-6) in formulae (I) and (II) represent a 1-2 bond (that is, a position-1 carbon-position-2 carbon bond), a 1-3 bond, a 1-5 bond, and a 1-6 bond between residues, respectively.

In one embodiment, in the acidic polysaccharide, a glycerol 3-phosphate group having a repeating structure in which repeating units represented by formula (I) are linked (e.g., in formula (II)), an average of about 1 per repeating unit (e.g., 1 (n = 1) or 0 for each repeating unit, and about 1 for each repeating unit in the entire weighted average) is added, but there is no limitation thereto.

<Others>

A particularly preferred embodiment of the method for producing an exopolysaccharide of the present embodiment is as follows, in which culture is performed for a specific time using a specific exopolysaccharide-producing bacterium.

A method for producing an exopolysaccharide, the method including a step of:

treating any exopolysaccharide-producing bacterium selected from a bacterium belonging to *Lactobacillus delbrueckii,* a bacterium belonging to *Lactococcus lactis,* a bacterium belonging to *Streptococcus thermophilus,* and a bacterium belonging to *Bifidobacterium breve* under a condition that a lag phase is prolonged by 10% or more; and culturing the treated exopolysaccharide-producing bacterium in a medium for 4 to 48 hours to produce an exopolysaccharide.

In this embodiment, various conditions described herein can be taken other than the bacterium and the culture time. In this embodiment, the culture time may be 4 to 36 hours, 4 to 24 hours, 6 to 48 hours, 6 to 36 hours, 6 to 24 hours, 8 to 48 hours, 8 to 36 hours, 8 to 24 hours, 12 to 48 hours, 12 to 36 hours, 12 to 24 hours, 14 to 48 hours, 14 to 36 hours, 14 to 24 hours, 16 to 36 hours, or 16 to 24 hours.

[Exopolysaccharide-producing bacterium treated under condition that lag phase is prolonged, and composition containing same]

The present embodiment relates to a composition containing an exopolysaccharide-producing bacterium treated under a condition that a lag phase is prolonged by 10% or more, and an exopolysaccharide-producing bacterium having an ability to produce EPS increased by about 5% as compared with a conventional one. By performing treatment under the condition that a lag phase is prolonged by 10% or more, a bacterium having an ability to produce EPS increased by about 5% as compared with a conventional one is obtained.

<Exopolysaccharide-producing bacterium treated under condition that lag phase is prolonged by 10% or more>

The exopolysaccharide-producing bacterium of the present embodiment can be specified as follows by an ability to produce an exopolysaccharide (hereinafter, also referred to as EPS production ability) when a skimmed milk powder medium is used.

A bacterium belonging to the genus *Lactobacillus,* preferably a bacterium belonging to *Lactobacillus delbrueckii,* more preferably a bacterium belonging to *Lactobacillus delbrueckii* ssp. *bulgaricus,* and still more preferably *Lactobacillus delbrueckii* ssp. *bulgaricus* OLL1073R-1 (FERM BP-10741), having an EPS production ability of more than 125 mg/kg, preferably 150 mg/kg or more, more preferably 175 mg/kg or more, and still more preferably 300 mg/kg or more;

a bacterium belonging to the genus *Lactococcus,* preferably a bacterium belonging to *Lactococcus lactis* spp. *lactis,*

and more preferably *Lactococcus lactis* spp. *lactis* OLS3789 (NITE BP-1387), having an EPS production ability of 2.2 mg/kg or more, preferably 5 mg/kg or more, more preferably 10 mg/kg or more, and still more preferably 15 mg/kg or more;

a bacterium belonging to the genus *Streptococcus,* preferably a bacterium belonging to *Streptococcus thermophilus,* and more preferably *Streptococcus thermophilus* OLS 3290 (FERM BP-19638), having an EPS production ability of 69 mg/kg or more, preferably 72 mg/kg or more, more preferably 75 mg/kg or more, and still more preferably 80 mg/kg or more; and

a bacterium belonging to the genus *Bifidobacterium,* preferably a bacterium belonging to *Bifidobacterium breve,* and more preferably *Bifidobacterium breve* JCM1192T, having an EPS production ability of 4.2 mg/kg or more, preferably 7 mg/kg or more, more preferably 10 mg/kg or more, and more preferably 14 mg/kg or more.

Incidentally, in the present invention, when referring to a concentration or amount of EPS, it refers to a concentration or amount measured by a phenol-sulfuric acid method (Ziadi et al., BioMed Research International, (2018) Vol.2018, doi:10.1155/2018/1896240.) unless otherwise specified. Examples of detailed conditions for the phenol-sulfuric acid method are described in the section of Examples herein. A concentration or amount of EPS in a subject may be determined by converting a value measured by a method other than the phenol-sulfuric acid method into a measured value by the phenol-sulfuric acid method as necessary. As a method for measuring EPS other than the phenol-sulfuric acid method, a method for efficiently detecting EPS by sandwiching exopolysaccharides using lectin that specifically binds to EPS (WO 2022/220154 A) or the like is known.

In one embodiment, the exopolysaccharide-producing bacterium can be specified as follows by an ability to produce EPS when a skimmed milk powder medium is used.

*Lactobacillus delbrueckii* subsp. *bulgaricus* OLL205013 (NITE BP-02411) having an EPS production ability of 2.1 mg/kg or more, preferably 5 mg/kg or more, more preferably 7 mg/kg or more, and still more preferably 7.5 mg/kg or more;

*Lactobacillus delbrueckii* subsp. *bulgaricus* OLL1171 (NITE BP-01569) having an EPS production ability of 16.4 mg/kg or more, preferably 20 mg/kg or more, more preferably 24 mg/kg or more, and still more preferably 28 mg/kg or more; and

*Lactobacillus delbrueckii* OLL204989 (NITE BP-02874) having an EPS production ability of 7.7 mg/kg or more, preferably 20 mg/kg or more, more preferably 30 mg/kg or more, and still more preferably 40 mg/kg or more.

In one embodiment, the exopolysaccharide-producing bacterium can be specified as follows by an ability to produce EPS when a skimmed milk powder medium is used.

*Streptococcus thermophilus* OLS3618 (NITE BP-01815) having an EPS production ability of 4.4 mg/kg or more, preferably 6 mg/kg or more, more preferably 7.5 mg/kg or more, and more preferably 9 mg/kg or more.

The exopolysaccharide-producing bacterium of the present embodiment can be specified as follows by an ability to produce EPS when a synthetic medium is used:

a bacterium belonging to the genus *Lactobacillus,* preferably a bacterium belonging to *Lactobacillus delbrueckii,* more preferably a bacterium belonging to *Lactobacillus delbrueckii* ssp. *bulgaricus,* and still more preferably *Lactobacillus delbrueckii* ssp. *bulgaricus* OLL1073R-1, having an EPS production ability of 301 mg/kg or more, preferably 305 mg/kg or more, more preferably 310 mg/kg or more, and still more preferably 314 mg/kg or more; and

a bacterium belonging to the genus *Lactococcus,* preferably a bacterium belonging to *Lactococcus lactis* spp. *lactis,* and more preferably *Lactococcus lactis* spp. *lactis* OLS3789 (NITE BP-1387), having an EPS production ability of 32 mg/kg or more, preferably 45 mg/kg or more, more preferably 70 mg/kg or more, and still more preferably 80 mg/kg or more.

Incidentally, when a synthetic medium is used, the synthetic medium can be selected according to an exopolysaccharide-producing bacterium to be used.

In one embodiment, the exopolysaccharide-producing bacterium can be specified as follows by an ability to produce EPS when a synthetic medium is used.

*Lactobacillus delbrueckii* subsp. *bulgaricus* OLL205013 (NITE BP-02411) having an EPS production ability of 255 mg/kg or more, preferably 260 mg/kg or more, more preferably 270 mg/kg or more, and still more preferably 280 mg/kg or more

*Lactobacillus delbrueckii* subsp. *bulgaricus* OLL1171 (NITE BP-01569) having an EPS production ability of 209 mg/kg or more, preferably 240 mg/kg or more, more preferably 275 mg/kg or more, and still more preferably 305 mg/kg or more

*Lactobacillus delbrueckii* OLL204989 (NITE BP-02874) having an EPS production ability of 218 mg/kg or more, preferably 230 mg/kg or more, more preferably 240 mg/kg or more, and still more preferably 250 mg/kg or more

<Exopolysaccharide-producing bacterium having lag phase of specific length>

The exopolysaccharide-producing bacterium of the present embodiment can be specified as follows by the length of the lag phase when a skimmed milk powder medium is used.

A bacterium belonging to the genus *Lactobacillus,* preferably a bacterium belonging to *Lactobacillus delbrueckii,* more preferably a bacterium belonging to *Lactobacillus delbrueckii* ssp. *bulgaricus,* and still more preferably a bacterium belonging to *Lactobacillus delbrueckii* subsp. *bulgaricus* OLL 1171 (NITE BP-01569), in which the length of the lag phase is 152 minutes or more, preferably 400 minutes or more, more preferably 700 minutes or more, and still more preferably 1000 minutes or more;

a bacterium belonging to the genus *Lactococcus,* preferably a bacterium belonging to *Lactococcus lactis* spp. *lactis,* and more preferably *Lactococcus lactis* spp. *lactis* OLS3789 (NITE BP-1387), in which the length of the lag phase is 236 minutes or more, preferably 240 minutes or more, more preferably 244 minutes or more, and still more preferably 248 minutes or more;

a bacterium belonging to the genus *Streptococcus,* preferably a bacterium belonging to *Streptococcus thermophilus,* and more preferably *Streptococcus thermophilus* OLS3618 (NITE BP-01815), in which the length of the lag phase is 199 minutes or more, preferably 206 minutes or more, more preferably 212 minutes or more, and still more preferably 219 minutes or more; and

a bacterium belonging to the genus *Bifidobacterium,* preferably a bacterium belonging to *Bifidobacterium breve,* and more preferably *Bifidobacterium breve* JCM1192T, in which the length of the lag phase is 425 minutes or more, preferably 431 minutes or more, more preferably 436 minutes or more, and further preferably 442 minutes or more.

When an upper limit value is set in the lag phase from the viewpoint of fermented milk production and the like, the upper limit value is 1010 minutes or less, and preferably 800 or less.

Incidentally, in the present invention, when the exopolysaccharide-producing bacterium is specified by the length of the lag phase, unless otherwise specified, the length refers to a time required to change pH in an initial stage of culture by 0.2 when a target exopolysaccharide-producing bacterium is cultured using a medium containing a milk raw material. As the medium using a milk raw material, it is preferable to use a skimmed milk powder medium, and it is more preferable to use a 10% reduced skimmed milk medium (pH 6.5), regardless of which bacterium is used. The 10% reduced skimmed milk medium refers to a medium containing 10% (W/W) of a skimmed milk component (reduced skimmed milk) that has been sterilized by an appropriate means, for example, sterilized at 95°C. Composition of the skimmed milk powder is, for example, 1% by mass of fat, 34% by mass of protein, 54% by mass of lactose, 8% by mass of ash, and 96% by mass of non-fat milk solid content. The 10% reduced skimmed milk medium contains, for example, 5.4% by mass of lactose and 9.6% by mass of a non-fat milk solid content.

In addition, before culturing the target exopolysaccharide-producing bacterium using the medium containing a milk raw material, conditions of activation culture and the medium can be specifically performed according to the method described in Examples and the description in the section of <Ability to produce exopolysaccharide (EPS production ability) >.

In one embodiment, the exopolysaccharide-producing bacterium can be specified as follows by the length of the lag phase using a skimmed milk powder medium.

*Lactobacillus delbrueckii* ssp. *bulgaricus* OLL1073R-1 (FERM BP-10741) in which the length of the lag phase is 126 minutes or more, preferably 380 minutes or more, more preferably 630 minutes or more, and further preferably 900 minutes or more;

*Lactobacillus delbrueckii* subsp. *bulgaricus* OLL205013 (NITE BP-02411) in which the length of the lag phase is 89 minutes or more, preferably 102 minutes or more, more preferably 115 minutes or more, and further preferably 128 minutes or more; and

*Lactobacillus delbrueckii* OLL204989 (NITE BP-02874) in which the length of the lag phase is 99 minutes or more, preferably 100 minutes or more, more preferably 102 minutes or more, and further preferably 103 minutes or more

In one embodiment, the exopolysaccharide-producing bacterium can be specified as follows by the length of the lag phase when a skimmed milk powder medium is used.

*Streptococcus thermophilus* OLS3290 (FERM BP-19638) in which the length of the lag phase is 99 minutes or more, preferably 100 minutes or more, more preferably 102 minutes or more, and further preferably 103 minutes or more.

When an upper limit value is set in the lag phase from the viewpoint of fermented milk production and the like, the upper limit value is 1000 minutes or less, preferably 700 or less, and more preferably 300 or less.

The exopolysaccharide-producing bacterium of the present embodiment can be specified as follows by the length of the lag phase when a synthetic medium is used:

a bacterium belonging to the genus *Lactobacillus,* preferably a bacterium belonging to *Lactobacillus delbrueckii,* more

preferably a bacterium belonging to *Lactobacillus delbrueckii* ssp. *bulgaricus,* and still more preferably *Lactobacillus delbrueckii* subsp. *bulgaricus* OLL1073R-1 (FERM BP-10741), in which the length of the lag phase is 136 minutes or more, preferably 355 minutes or more, more preferably 574 minutes or more, and still more preferably 793 minutes or more; and

a bacterium belonging to the genus *Lactococcus,* preferably a bacterium belonging to *Lactococcus lactis* spp. *lactis,* and more preferably *Lactococcus lactis* spp. *lactis* OLS3789 (NITE BP-1387), in which the length of the lag phase is 198 minutes or more, preferably 191 minutes or more, more preferably 194 minutes or more, and still more preferably 196 minutes or more.

Incidentally, when a synthetic medium is used, the synthetic medium can be selected according to an exopolysaccharide-producing bacterium to be used.

When an upper limit value is set in the lag phase from the viewpoint of fermented milk production and the like, the upper limit value is 1000 minutes or less, preferably 700 or less, and more preferably 300 or less.

In one embodiment, the exopolysaccharide-producing bacterium can be specified as follows by the length of the lag phase when a synthetic medium is used.

*Lactobacillus delbrueckii* subsp. *bulgaricus* OLL205013 (NITE BP-02411) in which the length of the lag phase is 105 or more, preferably 129 minutes or more, more preferably 152 minutes or more, and still more preferably 176 minutes or more

*Lactobacillus delbrueckii* subsp. *bulgaricus* OLL1171 (NITE BP-01569) in which the length of the lag phase is 115 minutes or more, preferably 167 minutes or more, more preferably 219 minutes or more, and still more preferably 271 minutes or more

*Lactobacillus delbrueckii* OLL204989 (NITE BP-02874) in which the length of the lag phase is 89 minutes or more, preferably 91 minutes or more, more preferably 92 minutes or more, and still more preferably 93 minutes or more

When an upper limit value is set in the lag phase from the viewpoint of fermented milk production and the like, the upper limit value is 500 minutes or less, and preferably 300 minutes or less.

<Ability to produce exopolysaccharide (EPS production ability)>

In the present embodiment, the EPS production ability of the exopolysaccharide-producing bacterium when a skimmed milk powder medium is used can be defined as an EPS content (mg/kg) of a culture when a bacterial solution of $1.0 \times 10^4$ to $10 \times 10^{10}$ cfu/g is added to the skimmed milk powder medium so as to be 1% by mass and cultured (main culture) at an optimum temperature for 24 hours. The optimum temperature is around 30°C for *Lactococcus lactis* spp. *lactis,* and 37 to 43°C for other lactic acid bacteria. The skimmed milk powder medium refers to a 10% skimmed milk powder medium unless otherwise specified.

An amount of bacteria added is preferably an amount to be $2 x 10^6$ to $5 \times 10^7$ cfu/g, more preferably an amount to be $4 \times 10^6$ to $4 \times 10^7$ cfu/g, and still more preferably an amount to be $1 \times 10^7$ to $2 \times 10^7$ cfu/g in terms of viable cell count with respect to a skimmed milk medium or an MRS medium.

The skimmed milk powder medium may be used by adding a fermentation accelerator as necessary. Specifically, inosinic acid is added for a bacterium belonging to the genus *Lactobacillus,* for example, a bacterium belonging to *L. delbrueckii,* a casein peptide is added for a bacterium belonging to the genus *Streptococcus,* for example, a bacterium belonging to *S. thermophilus,* inosinic acid is added for a bacterium belonging to the genus *Bifidobacterium,* for example, a bacterium belonging to *B. breve,* and a yeast extract is added for a bacterium belonging to the genus *Lactococcus,* for example, a bacterium belonging to *L. lactis,* at an effective concentration. When the EPS production ability of the exopolysaccharide-producing bacterium is evaluated, the skimmed milk powder medium does not contain formic acid, malic acid, fumaric acid, and salts thereof. In addition, as a raw material derived from milk to be used, a raw material in which lactose is not reduced by a lactose-degrading enzyme (a raw material in which a lactose-degrading enzyme is not contained) is used.

Incidentally, as necessary, it is preferable to perform activation culture before main culture. As the activation culture, it is preferable to perform culture at 37°C for 16 to 18 hours (preferably 16 hours) anaerobically in a medium suitable for a target exopolysaccharide-producing bacterium. At this time, an amount of bacteria to be subjected to the activation culture is preferably an amount to be $1 \times 10^4$ to $2 \times 10^8$ cfu/g, and more preferably an amount to be $1 \times 10^4$ to $1 \times 10^6$ cfu/g in terms of viable cell count with respect to the medium suitable for a target bacterium.

Examples of a medium for the activation culture include a skimmed milk powder medium or an MRS medium for a bacterium belonging to *L. delbrueckii* and *L. Lactis,* an MRS medium for a bacterium belonging to *L. lactis,* an M17 medium for a bacterium belonging to *S. thermophilus,* and a GAM medium for a bacterium belonging to *B. breve.*

The ability to produce EPS can also be defined as when a synthetic medium is used. Specifically, the ability to produce EPS can be defined as a content (mg/kg) of EPS of a culture when a lactic acid bacterium solution of $1.0 \times 10^4$ to

$10 \times 10^{10}$ cfu/g is added to a medium suitable for a target bacterium so as to be 1% by mass, and cultured at an optimum temperature for 24 hours. Examples of the synthetic medium include an MRS medium for a bacterium belonging to *L. delbrueckii,* an MRS medium for a bacterium belonging to *L. lactis,* an M17 medium for a bacterium belonging to S. *thermophilus,* and a GAM medium for a bacterium belonging to *B. breve.* When the EPS production ability of the exopolysaccharide-producing bacterium is evaluated, the synthetic medium does not contain components other than standard components.

<Composition>

A composition of the present embodiment can be used to utilize EPS as a functional ingredient (it may also be referred to as an active ingredient). EPS produced by lactic acid bacteria is known to have various functionalities such as an immunomodulatory action, an action as a prebiotic, an anticancer action, and an action of decreasing cholesterol levels (Non Patent Document 1, supra). Therefore, in one embodiment, a composition containing EPS can be used for utilizing EPS for immunomodulation, for use as a prebiotic, for treatment of cancer (risk reduction, prevention, treatment, and the like), and for control of cholesterol levels.

EPS produced by OLL1073R-1 has been recognized to have an NK cell-activating action (Patent Document 1, supra) and an anti-influenza effect (Patent Document 2, supra). Therefore, a composition containing OLL1073R-1 can be used for utilizing EPS produced by OLL1073R-1 for immunomodulation, for use as a prebiotic, for treatment of cancer (risk reduction, prevention, treatment, and the like), and for control of cholesterol levels.

The composition of the present embodiment may include a culture. The culture can contain a lactic acid bacterium and EPS.

Examples of the culture provided by the present embodiment are as follows.

A culture of a bacterium belonging to the genus *Lactobacillus,* preferably a bacterium belonging to *Lactobacillus delbrueckii,* more preferably a bacterium belonging to *Lactobacillus delbrueckii* ssp. *bulgaricus,* and still more preferably *Lactobacillus delbrueckii* ssp. *bulgaricus* OLL1073R-1, containing 300 mg/kg or more, preferably 304 mg/kg or more, more preferably 308 mg/kg or more, and still more preferably 312 mg/kg or more of EPS, preferably EPS produced by the bacterium;

a culture of a bacterium belonging to the genus *Lactococcus,* preferably a bacterium belonging to *Lactococcus lactis* spp. *lactis,* and more preferably *Lactococcus lactis* spp. *lactis* OLS3789, containing 2.2 mg/kg or more, preferably 5 mg/kg or more, more preferably 10 mg/kg or more, and still more preferably 15 mg/kg or more of EPS, preferably EPS produced by the bacterium;

a culture of a bacterium belonging to the genus *Streptococcus,* preferably a bacterium belonging to *Streptococcus thermophilus,* and more preferably *Streptococcus thermophilus* OLS 3290, containing 69 mg/kg or more, preferably 72 mg/kg or more, more preferably 75 mg/kg or more, and still more preferably 80 mg/kg or more of EPS, preferably EPS produced by the bacterium; and

a culture of a bacterium belonging to the genus *Bifidobacterium,* preferably a bacterium belonging to *Bifidobacterium breve,* and more preferably *Bifidobacterium breve* JCM1192T, containing 4.2 mg/kg or more, preferably 7 mg/kg or more, more preferably 10 mg/kg or more, and more preferably 14 mg/kg or more of EPS, preferably EPS produced by the bacterium.

Incidentally, as described above, the amount of EPS herein is described as an amount measured by the phenol-sulfuric acid method (due to nature of the measurement, polysaccharides other than EPS produced by bacterial cells, for example, polysaccharides derived from medium components may be contained) unless otherwise specified.

The composition may be in a form of a fermented food. Examples of the fermented food include fermented foods such as fermented milk, cheese, a fermented product using a vegetable raw material (soybean milk, almond milk, coconut milk, or the like) (e.g., vegetable raw material fermented yogurt), pickled vegetable, kimchi, soy sauce, and miso, and preferably fermented milk, cheese, and a fermented product using a vegetable raw material. The yogurt may be, for example, plain yogurt, hard yogurt, drink yogurt, soft yogurt, or frozen yogurt. The fermented product may contain various substances such as food raw materials and food additives used in the food field, and nutrient components and additives for culture, in addition to the above microorganisms and fermentation substrates.

[Method for producing fermented milk]

The present embodiment relates to a method for producing a fermented milk containing EPS, using an exopolysaccharide-producing bacterium treated so that a lag phase is prolonged by 10% or more or an exopolysaccharide-producing bacterium treated so that a concentration of EPS produced in a culture solution increases, as described above.

In one embodiment, the production method of the present embodiment includes a step of fermenting a raw material milk with one or two or more lactic acid-producing bacteria including exopolysaccharide-producing bacteria treated so that a lag phase is prolonged by 10% or more to obtain a fermented milk containing EPS. The exopolysaccharide-producing bacterium treated so that a lag phase is prolonged by 10% or more used in the present embodiment is the same as the "exopolysaccharide-producing bacterium treated under a condition that a lag phase is prolonged by 10% or more" in the section of "Exopolysaccharide-producing bacterium treated under condition that lag phase is prolonged by 10% or more, and composition containing same". In another embodiment, a step of fermenting a raw material milk with one or two or more lactic acid-producing bacteria including exopolysaccharide-producing bacteria treated so that a concentration of EPS produced in a culture solution increases to obtain a fermented milk containing EPS is included. The lactic acid bacterium treated so that a lag phase is prolonged by 10% or more in the present embodiment is the same as the treatment described in "Step of pretreating exopolysaccharide-producing bacterium" in the section of "Method for producing exopolysaccharide". The one or two or more lactic acid-producing bacteria may include, for example, a bacterium belonging to the genus *Streptococcus,* preferably a bacterium belonging to *Streptococcus thermophilus,* in addition to the treated exopolysaccharide-producing bacterium.

In the production method of the present embodiment, the content of the exopolysaccharide in the fermented milk after the fermentation step or the content of the exopolysaccharide in the obtained fermented milk is as follows.

When one or two or more lactic acid bacteria including a bacterium belonging to the genus *Lactobacillus,* preferably a bacterium belonging to *Lactobacillus delbrueckii,* more preferably a bacterium belonging to *Lactobacillus delbrueckii* ssp. *bulgaricus,* and still more preferably *Lactobacillus delbrueckii* ssp. *bulgaricus* OLL1073R-1, the content of the exopolysaccharide in the obtained fermented milk is 300 mg/kg or more, preferably 304 mg/kg or more, more preferably 308 mg/kg or more, and still more preferably 312 mg/kg or more;

when one or two or more lactic acid bacteria including a bacterium belonging to the genus *Lactococcus,* preferably a bacterium belonging to *Lactococcus lactis* spp. *lactis,* and more preferably *Lactococcus lactis* spp. *lactis* OLS3789, the content of the exopolysaccharide in the obtained fermented milk is 2.2 mg/kg or more, preferably 5 mg/kg or more, more preferably 10 mg/kg or more, and still more preferably 15 mg/kg or more;

when one or two or more lactic acid bacteria including a bacterium belonging to the genus *Streptococcus,* preferably a bacterium belonging to *Streptococcus thermophilus,* and more preferably *Streptococcus thermophilus* OLS 3290, the content of the exopolysaccharide in the obtained fermented milk is 69 mg/kg or more, preferably 72 mg/kg or more, more preferably 75 mg/kg or more, and still more preferably 80 mg/kg or more; and

when one or two or more lactic acid bacteria including a bacterium belonging to the genus *Bifidobacterium,* preferably a bacterium belonging to *Bifidobacterium breve,* and more preferably *Bifidobacterium breve* JCM1192T, the content of the exopolysaccharide in the obtained fermented milk is 4.2 mg/kg or more, preferably 7 mg/kg or more, more preferably 10 mg/kg or more, and more preferably 14 mg/kg or more.

In another embodiment, the production method of the present embodiment includes a step of obtaining EPS from an exopolysaccharide-producing bacterium treated so that a lag phase is prolonged by 10% or more, and a step of adding the obtained EPS to a fermented milk. In still another embodiment, a step of obtaining EPS from an exopolysaccharide-producing bacterium treated so that a concentration of EPS produced in a culture solution increases, and a step of adding the obtained EPS to a fermented milk are included.

The raw material milk used for obtaining a fermented milk includes a dairy product. Examples of dairy products that can be used include animal milk such as cattle and goats, sterilized milk, skimmed milk, whole milk powder, skimmed milk powder, whole condensed milk, skimmed condensed milk, cream, butter, buttermilk, and whey. Milk proteins include a milk protein concentrate (MPC), a whey protein concentrate (WPC), a whey protein isolate (WPI), $\alpha$-lactalbumin ($\alpha$-La), $\beta$-lactoglobulin ($\beta$-Lg), a thermally denatured whey protein, and an enzyme-treated whey protein.

The raw material milk may contain other raw materials. Examples of the other raw materials include sugar, sweeteners, saccharides, flavors, and water. In addition, examples of the other raw materials include gelling agents, thickeners, and stabilizers such as gelatin, agar, pectin, carboxymethyl cellulose (CMC), xanthan gum, carrageenan, tamarind seed gum, guar gum, gum arabic, locust bean gum, gellan gum, soybean polysaccharides, and processed starch as necessary.

In one embodiment, a raw material milk in which a level of lactose is not reduced is used. Although production of EPS can be increased by lowering the level of lactose to a relatively low level in a raw material milk used for a medium, the present embodiment can exclude an embodiment in which a raw material milk in which lactose has been previously reduced by a lactose-degrading enzyme is used.

The method for producing a fermented milk of the present embodiment may include a homogenization step, a sterilization step, a step of filling a container with a fermented milk, and the like.

[Method for Improving ability to produce exopolysaccharide of exopolysaccharide-producing bacterium]

The present embodiment relates to a method for improving an ability to produce an exopolysaccharide of a lactic acid bacterium, the method including performing treatment under a condition that a lag phase is prolonged, and a method for improving an ability to produce an exopolysaccharide of a lactic acid bacterium, the method including performing any treatment selected from the group consisting of heat (high-heat) treatment, osmotic pressure (high-osmotic pressure) treatment, and pH (high-pH) treatment.

Regarding the lactic acid bacterium applicable to the present embodiment, the applicable lactic acid bacterium described in the section of Method for producing exopolysaccharide can be similarly used in the present embodiment.

This embodiment is based on a finding of the present inventors that, in culture of a lactic acid bacterium, subjecting an exopolysaccharide-producing bacterium to a condition that a lag phase is prolonged by 10% or more before culture increases a concentration of EPS produced in a culture solution, and that there is a positive correlation between a length of the lag phase and the concentration of EPS in the culture solution (see Figs. 1 to 6).

EXAMPLES

[Example 1: Increase in production amount of polysaccharides by *L. delbrueckii* by heat treatment (skimmed milk powder medium)]

For four strains of *L. delbrueckii* including OLL1073R-1, an influence of heat treatment of bacterial cells on production of polysaccharides during skimmed milk powder fermentation was verified.

<Method>

- Investigational strains:

  Lactobacillus delbrueckii subsp. bulgaricus OLL1073R-1 (FERM BP-10741)
  Lactobacillus delbrueckii subsp. bulgaricus OLL205013 (NITE BP-02411),
  Lactobacillus delbrueckii subsp. bulgaricus OLL1171 (NITE BP-01569),
  *Lactobacillus delbrueckii* OLL204989 (NITE BP-02874)

- Heat treatment conditions: 250 μL of each bacterial solution (a solution obtained by culturing bacteria in a predetermined medium containing protein components (skimmed milk powder, lactose monohydrate, yeast extract, fish extract, SMO (SUNSOFT Q17S), and water). Cell count: about $5.8 \times 10^9$ cfu/g.) was placed in a 1.5 mL tube, and heated at 60°C for 10 minutes with a heat block. The heat-treated bacterial solution was frozen and stored as necessary.
- Fermentation medium: A 10% reduced skimmed milk medium (a 10% by mass aqueous solution of a skimmed milk powder (1% by mass of fat, 34% by mass of protein, 54% by mass of lactose, 8% by mass of ash, and 96% by mass of non-fat milk solid content) (manufactured by Meiji Co., Ltd.). In the medium, 5.4% by mass of lactose content, and 9.6% by mass of non-fat milk solid content) to which inosinic acid was added so as to have a final concentration of 1 mM, sterilized at 95°C, pH 6.5.
  The 10% reduced skimmed milk medium means a medium (reduced skimmed milk) containing a skimmed milk component at 10% (W/W).
- Fermentation conditions: 1% of a bacterial solution (frozen bacteria) was inoculated into the fermentation medium, fermentation was performed at 40°C, and fermentation was stopped after 24 hours by placing the fermentation medium on ice.
- Method for measuring polysaccharide concentration: phenol-sulfuric acid method

Pretreatment:

1 mL of a 100 w/v% trichloroacetic acid solution was added to 10 g of the fermented product after 24 hours of fermentation, and the mixture was stirred, and then centrifuged at 13400 g and 4°C for 10 minutes. The supernatant was collected, and 5 mL of a 10%w/v trichloroacetic acid solution was added. The mixture was stirred, and then centrifuged at 13400 g and 4°C for 10 minutes. The supernatant was collected, and 100% ethanol in an amount 2 times that of the supernatant was added and mixed, and then the mixture was allowed to stand at 10°C overnight. After centrifugation at 13400 g and 4°C for 20 minutes, the supernatant was discarded, 9 mL of 100% ethanol was added, and the mixture was

allowed to stand in a refrigerator for 20 minutes, which was repeated twice. The mixture was centrifuged at 13400 g and 4°C for 20 minutes, the supernatant was discarded, 10 mL of purified water was added, and the mixture was completely dissolved using a shaker.

Measurement method:

500 μL of the pretreatment solution was transferred to a middle test tube, 500 μL of a 5% (w/v) phenol aqueous solution was added thereto, and the mixture was vortexed for 5 seconds. 2.5 mL of concentrated sulfuric acid was added and vortexed immediately for 10 seconds. After being allowed to stand at room temperature for 20 to 40 minutes, the whole amount was transferred to a cuvette, and an OD of 490 nm was measured. A calibration curve was prepared using the result of D(+) glucose measured as a standard, and a value obtained by multiplying the concentration calculated from the calibration curve by 0.9 was taken as the polysaccharide concentration. In the following experiments, unless otherwise specified, the polysaccharide concentration was measured by this method after the pretreatment described above.

• Method for measuring pH:

pH was continuously measured by attaching a pH sensor (pH Sencer SE 555, manufactured by Knick Elektronische Messgeräte GmbH & Co. KG.) to a pH monitoring device (manufactured by HORIBA, Ltd.), and performing fermentation with the electrode inserted into the fermented product. In the following experiments, pH was measured by this method unless otherwise specified.

<Results>

The EPS content of each fermented product after 24 hours of fermentation is shown in the following table and Fig. 1. As shown in the following table, it was revealed that the polysaccharide content after 24 hours of fermentation is increased in all of the four strains of *L. delbrueckii* by performing fermentation after heat treatment at 60°C for 10 minutes.

In addition, the delay rate (time (minutes) required to change pH in the initial stage of culture by 0.2, that is, the delay rate of the time until pH of the fermentation medium changed from pH 6.5 to pH 6.3 in the present Example) in the lag phase was as shown in the following table.

Delay rate in lag phase = [(Time (minutes) required for treated lactic acid bacterium to change pH in initial stage of culture by 0.2) - (Time (minutes) required for untreated lactic acid bacterium to change pH in initial stage of culture by 0.2)]/(Time (minutes) required for untreated lactic acid bacterium to change pH in initial stage of culture by 0.2)

[Table 1]

| Lactic acid bacterium starter | Polysaccharide content (mg/kg) | |
|---|---|---|
| | Without heat treatment | With heat treatment |
| OLL1073R-1 | 28.3 | 163.8 |
| OLL205013 | 2.0 | 7.8 |
| OLL1171 | 15.6 | 30.0 |
| OLL204989 | 7.3 | 42.9 |

| Lactic acid bacterium Starter | Lag phase (time (minutes) required to pH 6.3 or less) | | |
|---|---|---|---|
| | Without heat treatment | With heat treatment | Delay (%) |
| OLL1073R-1 | 85 | 925 | 988 |
| OLL205013 | 85 | 135 | 59 |
| OLL1171 | 145 | 1020 | 603 |
| OLL204989 | 95 | 105 | 11 |

[Example 2: Increase in production amount of polysaccharides by *L. delbrueckii* by heat treatment (synthetic medium)]

In Example 1, yogurt fermentation was assumed, and a skimmed milk powder medium was used. In Example 2, assuming that a polysaccharide produced by a lactic acid bacterium is produced and added as a material to various products, the production amount of polysaccharides was verified for 4 strains of *L. delbrueckii* when a synthetic medium (MRS broth) was used.

<Method>

- Investigational strains: OLL1073R-1, OLL205013, OLL1171, and OLL204989
- Heat treatment conditions: the same as in Example 1
- Fermentation medium: MRS broth (Difco), pH 6.1

[Table 2-1]

| Approximate Formula Per Liter | |
|---|---|
| Proteose Peptone No.3 | 10.0 g |
| Beef Extract | 10.0 g |
| Yeast Extract | 5.0 g |
| Dextrose | 20.0 g |
| Polysorbate 80 | 1.0 g |
| Ammonium Citrate | 2.0 g |
| Sodium Acetate | 5.0 g |
| Magnesium Sulfate | 0.1 g |
| Manganese Sulfate | 0.05 g |
| Dipotassium Phosphate | 2.0 g |

- Fermentation conditions: 1% of frozen bacteria were inoculated into the fermentation medium, fermentation was performed at 40°C, and fermentation was stopped after 24 hours by placing the fermentation medium on ice.
- Method for measuring polysaccharide concentration: phenol-sulfuric acid method (described in Example 1)

<Results>

The EPS content of each fermented product after 24 hours of fermentation is shown in the following table and Fig. 2. It was revealed that the polysaccharide content after 24 hours of fermentation is increased also in the synthetic medium for 4 strains of *L. delbrueckii* by performing fermentation after heat treatment at 60°C for 10 minutes. In addition, the delay rate (time (minutes) required to change pH in the initial stage of culture by 0.2, that is, the delay rate of the time until pH of the fermentation medium changed from pH 6.1 to pH 5.9 in the present Example) in the lag phase was as shown in the following table.

[Table 2-2]

| Lactic acid bacterium starter | Polysaccharide content (mg/kg) | |
|---|---|---|
| | Without heat treatment | With heat treatment |
| OLL1073R-1 | 287.0 | 314.8 |
| OLL205013 | 242.9 | 284.9 |
| OLL1171 | 199.8 | 308.2 |
| OLL204989 | 208.2 | 252.2 |

| Lactic acid bacterium starter | Lag phase (time (minutes) required to pH 5.9 or less) | | |
|---|---|---|---|
| | Without heat treatment | With heat treatment | Delay (%) |
| OLL1073R-1 | 130 | 835 | 542 |
| OLL205013 | 10 | 185 | 85 |
| OLL1171 | 110 | 285 | 159 |

(continued)

| Lactic acid bacterium starter | Lag phase (time (minutes) required to pH 5.9 or less) | | |
|---|---|---|---|
| | Without heat treatment | With heat treatment | Delay (%) |
| OLL204989 | 85 | 95 | 12 |

[Example 3: Influence of heat treatment on production of polysaccharides of lactic acid bacterium species used in fermented milk]

For *S. thermophilus* and *B. breve,* which are bacterial species used for a fermented milk other than *L. delbrueckii,* an influence of heat treatment of bacterial cells on production of polysaccharides during fermentation was verified. Incidentally, two strains (*Streptococcus thermophilus* OLS3618 (NITE BP-01815), *Streptococcus thermophilus* OLS 3290 (FERM BP-19638)) of *S. thermophilus* were used, and one reference strain ($JCM1192^T$) of *B. breve* was used.

<Method>

- Activation medium; M17 broth (Gifco) for *S. thermophilus,* and GAM broth (Nissui Pharmaceutical Co., Ltd.) for *B. breve.*

[Table 3-1]

| M17 Broth | |
|---|---|
| | g (in 950 ml) |
| Pancreatic Digest of Casein | 5 |
| Soy Peptone | 5 |
| Beef Extract | 5 |
| Yeast Extract | 2.5 |
| Ascorbic Acid | 0.5 |
| Magnesium Sulfate | 0.25 |
| Disodium-β-glycerophosphate | 19 |
| Add 50 mL 10% lactose solution | |

GAM Broth

| | g (in 1 L) |
|---|---|
| Peptone | 10 |
| Soy Peptone | 3 |
| Proteose Peptone | 10 |
| Digested Serum Powder | 13.5 |
| Yeast Extract | 5 |
| Meat Extract | 2.2 |
| Liver Extract | 1.2 |
| Glucose | 3 |
| Potassium Dihydrogen Phosphate | 2.5 |

(continued)

| | g (in 1 L) |
|---|---|
| Sodium Chloride | 3 |
| Soluble Starch | 5 |
| L-Cysteine Hydrochloride | 0.3 |
| Sodium Thioglycolate | 0.3 |

- Activation culture conditions: 1% of frozen bacteria were added to the activation medium, and static culture was performed at 37°C for 16 hours, then 1% of the activation solution was added to the activation medium, and static culture was performed at 37°C for 16 hours.
- Heat treatment conditions: 250 μL of the activation solution was placed in a 1.5 mL tube, and heated at 60°C for 10 minutes with a heat block.
- Fermentation medium: A 10% reduced skimmed milk medium (sterilization at 95°C) was adjusted, and a casein peptide CMA500 (casein proteolytic product, trade name: Hyvital Casein CMA 500 Protein Hydrolysate, manufacturer: FrieslandCampina Domo, standard component value: protein: 86.4%, ash: 6.0%, moisture: <5%, fat: <0.1%, lactose: <0.1%, average molecular weight: 267 daltons, degradation method: enzymatic degradation) was added so as to have a final concentration of 0.1% during fermentation of *S. thermophilus,* and inosinic acid was added so as to have a final concentration of 1 mM during fermentation of *B. breve,* pH 6.5.
- Fermentation conditions: 1% of the activation solution was inoculated into the fermentation medium, fermentation was performed at 40°C, and fermentation was stopped at 24 hours.
- Method for measuring polysaccharide concentration: phenol-sulfuric acid method (described in Example 1)

<Results>

The EPS content of each fermented product after 24 hours of fermentation is shown in the following table. Also for two strains of *S. thermophilus* and one reference strain of *B. breve,* it was revealed that the polysaccharide content after 24 hours of fermentation is increased by performing fermentation after heat treatment at 60°C for 10 minutes. In addition, the delay rate (time (minutes) required to change pH in the initial stage of culture by 0.2, that is, the delay rate of the time until pH of the fermentation medium changed from pH 6.5 to pH 6.3 in the present Example) in the lag phase was as shown in the following table.

[Table 3-2]

| Lactic acid bacterium species | Name of lactic acid bacterium | Polysaccharide content (mg/kg) | |
|---|---|---|---|
| | | Without heat treatment | With heat treatment |
| *S.thermophilus* | OLS3618 | 4.2 | 9.4 |
| *S.thermophilus* | OLS3290 | 65.9 | 82.9 |
| *B.breve* | JCM1192$^T$ | 4.0 | 14.7 |

| Lactic acid bacterium species | Name of lactic acid bacterium | Lag phase (time (minutes) required to pH 6.3 or less) | | |
|---|---|---|---|---|
| | | Without heat treatment | With heat treatment | Delay (%) |
| *S.thermophilus* | OLS3618 | 190 | 230 | 21 |
| *S.thermophilus* | OLS3290 | 55 | 65 | 18 |
| *B.breve* | JCM1192$^T$ | 405 | 465 | 15 |

[Example 4: Increase in production amount of polysaccharides by *L. delbrueckii* due to difference in heat treatment conditions (skimmed milk powder medium)]

<Method>

OLL1073R-1 was heat-treated at 60°C for 3 minutes, 60°C for 5 minutes, 60°C for 10 minutes, 80°C for 1 minute,

and 80°C for 3 minutes, and the viable cell count after treatment was measured. Then, 1% of the heat-treated OLL1073R-1 was inoculated into a 10% skimmed milk powder medium (1 mM inosinic acid added, pH 6.5) sterilized at 95°C, and fermentation was performed at 40°C. Sampling was performed after 24 hours of fermentation, and measurement of viable cell count after fermentation and EPS concentration (phenol-sulfuric acid method, described in Example 1) was performed.

<Results>

The results are shown in the following table and Fig. 3. Even when heat treatment was performed at 60°C for 3 minutes or 60°C for 5 minutes, the viable cell count immediately after heat treatment decreased, but the EPS concentration after 24 hours of fermentation was improved as compared with a case without heat treatment. After heat treatment at 60°C for 10 minutes or 80°C for 1 minute, a significant decrease in viable cell count was confirmed, but the EPS concentration was improved as compared with a case without heat treatment.

The polysaccharide concentration per log10 (viable cell count) after 24 hours of fermentation under conditions of 60°C for 3 minutes, 60°C for 5 minutes, 60°C for 10 minutes, and 80°C for 1 minute was improved as compared with a case without heat treatment.

In addition, the delay rate (time (minutes) required to change pH in the initial stage of culture by 0.2, that is, the delay rate of the time until pH of the fermentation medium changed from pH 6.5 to pH 6.3 in the present Example) in the lag phase under conditions of 60°C for 3 minutes, 60°C for 5 minutes, 60°C for 10 minutes, and 80°C for 1 minute in which an increase in polysaccharide concentration was observed was as shown in the following table.

[Table 4]

| Heating conditions | Viable cell count after heat treatment (cfu/g) | Viable cell count after 24 hours of fermentation (cfu/g) | Logl0 (viable cell count after 24 hours of fermentation) | Polysaccharide concentration (mg/kg) | Polysaccharide concentration/log10 (viable cell count) |
|---|---|---|---|---|---|
| Without heat treatment | 1.3.E + 07 | 2.3.E + 07 | 7.4 | 102.3 | 13.9 |
| 60°C for 3 minutes | 1.3.E + 06 | 6.3.E + 07 | 7.8 | 153.9 | 19.7 |
| 60°C for 5 minutes | 4.7.E + 04 | 8.1.E + 08 | 8.9 | 182.3 | 20.5 |
| 60°C for 10 minutes | < 100 | 6.2.E + 08 | 8.8 | 169.6 | 19.3 |
| 80°C for 1 minute | 5.2.E + 05 | 5.0.E + 07 | 7.7 | 156.7 | 20.4 |
| 80°C for 3 minutes | < 100 | < 100 | - | 0.8 | - |

| Heating conditions | Time (minutes) to pH 6.3 or less | Delay (%) |
|---|---|---|
| Without heat treatment | 120 | - |
| 60°C for 3 minutes | 300 | 150 |
| 60°C for 5 minutes | 485 | 304 |
| 60°C for 10 minutes | 850 | 608 |
| 80°C for 1 minute | 290 | 142 |
| 80°C for 3 minutes | - | |

[Example 5: Increase in production amount of polysaccharides by *L. delbrueckii* by salt (osmotic pressure) treatment (skimmed milk powder medium)]

<Method>

OLL1073R-1 was mixed with frozen bacteria so as to have an NaCl final concentration of 0.8%, 5%, or 10%, and the mixture was allowed to stand (high-osmotic pressure treatment) at 37°C for 3 hours or 6 hours. Then, 2% of the treated OLL1073R-1 bacterial solution was inoculated into a 10% skimmed milk powder medium (1 mM inosinic acid added, pH 6.5) sterilized at 95°C, and fermentation was performed at 40°C. Sampling was performed after 24 hours of fermentation, and measurement of viable cell count after fermentation and measurement of EPS concentration (phenol-sulfuric acid method, described in Example 1) were performed.

<Results>

The results are shown in the following table and Fig. 4. By NaCl treatment, the EPS concentration after 24 hours of fermentation tended to be improved as the NaCl treatment concentration increased and as the treatment time was longer. The same was true for the polysaccharide concentration per log10 (viable cell count).

[Table 5]

| Stress conditions | | Viable cell count after treatment (cfu/g) | Viable cell count after 24 hours of fermentation (cfu/g) | Logl0 (viable cell count after 24 hours of fermentation) | Polysaccharide concentration (mg/kg) | Polysaccharide concentration/log 10 (viable cell count) |
|---|---|---|---|---|---|---|
| 37°C3 H | NaCl 0.8% | 1.7.E + 06 | 3.1.E + 08 | 8.5 | 105.4 | 12.4 |
| | NaCl 5% | 2.5.E + 06 | 2.1.E + 08 | 8.3 | 129.9 | 15.6 |
| | NaCl 10% | 2.7.E + 05 | 4.6.E + 08 | 8.7 | 127.4 | 14.7 |
| 37°C6 H | NaCl 0.8% | 8.9.E + 05 | 3.1.E + 08 | 8.5 | 95.3 | 11.2 |
| | NaCl 5% | 8.1.E + 05 | 4.3.E + 08 | 8.6 | 131.4 | 15.2 |
| | NaCl 10% | 4.1.E + 04 | 1.8.E + 08 | 8.3 | 157.3 | 19.1 |

| Salt treatment conditions | | Time (minutes) to pH 6.3 or less |
|---|---|---|
| 37°C 3H | NaCl 0.8% | 240 |
| | NaCl 5% | 215 |
| | NaCl 10% | 385 |
| 37°C 6H | NaCl 0.8% | 255 |
| | NaCl 5% | 280 |
| | NaCl 10% | 490 |

[Example 6: Increase in production amount of polysaccharides by *L. delbrueckii* by salt (osmotic pressure) treatment (skimmed milk powder medium)]

<Method>

OLL1073R-1 was mixed with frozen bacteria so as to have an NaCl final concentration of 0%, 0.8%, 5%, or 10%, and the mixture was allowed to stand (high-osmotic pressure treatment) at 37°C for 3 hours. Then, 4% (2% as bacteria) of the treated OLL1073R-1 bacterial solution was inoculated into a 10% skimmed milk powder medium (1 mM inosinic acid added, pH 6.5) sterilized at 95°C, and fermentation was performed at 40°C. Sampling was performed after 24 hours of fermentation, and measurement of viable cell count after fermentation and measurement of EPS concentration (phenol-sulfuric acid method, described in Example 1) were performed.

<Results>

As shown in the following table and Fig. 5, it was revealed that the polysaccharide content after 24 hours of fermentation is increased by performing fermentation after NaCl treatment. In addition, the delay rate (time (minutes) required to change pH in the initial stage of culture by 0.2, that is, the delay rate of the time until pH of the fermentation medium changed from pH 6.5 to pH 6.3 in the present Example) in the lag phase was as shown in the following table.

[Table 6]

| Stress conditions | | Viable cell count after treatment (cfu/g) | Viable cell count after 24 hours of fermentation (cfu/g) | Logl0 (viable cell count after 24 hours of fermentation) | Polysaccharide concentration (mg/kg) | Polysaccharide concentration/log10 (viable cell count) |
|---|---|---|---|---|---|---|
| Without treatment | | n.d. | 1.5E + 07 | 7.2 | 125.6 | 17.5 |
| 37°C 3H | NaCl 0.8% | 7.1E + 06 | 2.1E + 07 | 7.3 | 132.6 | 18.1 |
| | NaCl 5% | 8.5E + 06 | 2.0E + 06 | 6.3 | 136.2 | 21.6 |
| | NaCl 10% | 1.5E + 06 | 2.4E + 08 | 8.4 | 165.0 | 19.7 |

| Stress conditions | | Time (minutes) to pH 6.3 or less | Delay (%) |
|---|---|---|---|
| Without treatment | | 110 | - |
| 37°C 3H | NaCl 0.8% | 170 | 55 |
| | NaCl 5% | 160 | 45 |
| | NaCl 10% | 310 | 182 |

[Example 7: Increase in production amount of polysaccharides by *L. delbrueckii* by high-pH treatment]

<Method>

0.3% of OLL1073R-1 was added to a medium described in the following table (After autoclaving, filter-sterilized 2 mol/L DL-malic acid was added to the medium at 0.2% (final 4 mM)), and the culture initial pH was adjusted to pH 6.0 (control, without pH adjustment), pH 6.7 with potassium carbonate, and pH 6.9. The medium was cultured at 37°C, pH 5.4 for 24 hours while being neutralized with potassium carbonate.

[Table 7-1]

| | Blending ratio |
|---|---|
| | % |
| Skimmed milk powder | 4 |
| Anhydrous crystalline glucose | 7 |
| Beer yeast extract MEAST P2G | 0.5 |
| Fish extract Bacterio-N-KN | 1 |
| Emulsifier SUNSOFT Q17S | 0.05 |
| Antifoaming agent Awa Break L-01 | 0.1 |
| Total | 100 |

Sampling was performed after 24 hours of fermentation, and measurement of viable cell count after fermentation and measurement of EPS concentration (phenol-sulfuric acid method, described in Example 1) were performed.

<Results>

As shown in Fig. 6, it was revealed that when the initial pH is higher than usual, the polysaccharide content after 24 hours of fermentation is increased. In addition, the delay rate (time (minutes) required to change pH in the initial stage of culture by 0.2 in the lag phase was as shown in the following table.

[Table 7-2]

| Initial pH | Time (minutes) to ΔpH=0.2 | Delay (%) |
|---|---|---|
| 6.0 | 90 | - |
| 6.7 | 120 | 33 |
| 6.9 | 120 | 33 |

[Example 8: Increase in production amount of polysaccharides by *Lactococcus lactis* spp. *lactis* by heat treatment (MRS medium)]

<Method>

1% of frozen bacteria of *Lactococcus lactis* spp. *lactis* OLS3789 (NITE BP-1387) were added to an MRS broth, and static culture was performed at 37°C for 16 hours, then 1% of the activation solution was added to an activation medium (MRS), and static culture was performed at 37°C for 16 hours (activation culture).

250 μL of the activation culture solution was placed in a 1.5 mL tube, and heat-treated at 60°C for 1 minute with a heat block. 1% of the heat-treated bacterial solution of OLS3789 was inoculated into the MRS broth, fermentation was performed at 30°C, and fermentation was stopped after 24 hours by placing the MRS broth on ice. The polysaccharide concentration was measured according to the phenol-sulfuric acid method, described in Example 1.

<Results>

The results are shown in the following table. By heat treatment at 60°C for 1 minute, an increase in polysaccharide concentration was observed after 24 hours of fermentation. In addition, the delay rate (time (minutes) required to change pH in the initial stage of culture by 0.2, that is, the delay rate of the time until pH of the fermentation medium changed from pH 6.1 to pH 5.9 in the present Example) in the lag phase due to the heat treatment was as shown in the following table.

[Table 8]

| Heat stress | Polysaccharide concentration (mg/kg) | Time to pH 5.9 or less | Delay (%) |
|---|---|---|---|
| Without treatment | 31.1 | 180 | - |
| 60°C for 1 minute | 83.2 | 200 | 11 |

[Example 9: Increase in production amount of polysaccharides by *Lactococcus lactis* spp. *lactis* by salt (osmotic pressure) treatment (skimmed milk powder medium)]

<Method>

1% of frozen bacteria of *Lactococcus lactis* spp. *lactis* OLS3789 (NITE BP-1387) were added to an MRS broth, and static culture was performed at 37°C for 16 hours, then 1% of the activation solution was added to an activation medium (MRS), and static culture was performed at 37°C for 16 hours (activation culture).

The activation culture solution was mixed with frozen bacteria so as to have an NaCl final concentration of 2%, and the mixture was allowed to stand at 37°C for 3 hours. Thereafter, 2% (1% as bacteria) of the salt-treated bacterial solution of OLS3789 was inoculated into a medium prepared by adding 0.1% MEAST P2G (yeast extract) to a 10% skimmed milk powder medium, and fermentation was performed at 30°C, and after 24 hours, fermentation was stopped by placing the medium on ice. The polysaccharide concentration was measured according to the phenol-sulfuric acid method, Example 1.

<Results>

The results are shown in the following table. By salt treatment for 3 hours, an increase in polysaccharide concentration was observed after 24 hours of fermentation. In addition, the delay rate (time (minutes) required to change pH in the initial stage of culture by 0.2, that is, the delay rate of the time until pH of the fermentation medium changed from pH 6.5 to pH 6.3 in the present Example) in the lag phase due to the heat treatment was as shown in the following table.

[Table 9]

| Salt | Polysaccharide concentration (mg/kg) | Time (minutes) to pH 6.3 or less | Delay (%) |
|---|---|---|---|
| Without treatment | 2.1 | 225 | - |
| NaCl 2% 37°C 3H | 15.8 | 250 | 11 |

[Example 10: EPS production during fermentation of heat-treated *L. delbrueckii* (skimmed milk powder medium)]

For *L. delbrueckii,* an influence of heat treatment of bacterial cells on production of polysaccharides during skimmed milk powder fermentation was verified.

<Method>

- Investigational strains:
  Lactobacillus delbrueckii subsp. bulgaricus OLL1073R-1 (FERM BP-10741)
- Heat treatment conditions: 250 μL of each bacterial solution (a solution obtained by culturing bacteria in a predetermined medium containing protein components (skimmed milk powder, lactose monohydrate, yeast extract, fish extract, SMO (SUNSOFT Q17S), and water). Cell count: about $5.8 \times 10^9$ cfu/g.) was placed in a 1.5 mL tube, and heated at 60°C for 5 minutes and 80°C for 1 minute with a heat block. The heat-treated bacterial solution was frozen and stored as necessary.
- Fermentation medium: A 10% reduced skimmed milk medium (a 10% by mass aqueous solution of a skimmed milk powder (1% by mass of fat, 34% by mass of protein, 54% by mass of lactose, 8% by mass of ash, and 96% by mass of non-fat milk solid content) (manufactured by Meiji Co., Ltd.). In the medium, 5.4% by mass of lactose content, and 9.6% by mass of non-fat milk solid content) to which inosinic acid was added so as to have a final concentration of 1 mM, sterilized at 95°C, pH 6.5.
  The 10% reduced skimmed milk medium means a medium (reduced skimmed milk) containing a skimmed milk component at 10% (W/W).
- Fermentation conditions: 1% of a bacterial solution (frozen bacteria) was inoculated into the fermentation medium, fermentation was performed at 40°C, and fermentation was stopped after 24 hours by placing the fermentation medium on ice. In addition, sampling was also performed at the time point of pH 5.2 and at the time point of pH 4.6.
- Method for measuring polysaccharide concentration: described in Example 1
- Method for measuring pH: described in Example 1

<Results>

A delay in the lag phase was observed in any of heating at 60°C for 5 minutes and heating at 80°C for 1 minute (Fig. 7).

Even when heat treatment was performed at 60°C for 5 minutes, the viable cell count immediately after heat treatment did not decrease (Fig. 8), but the EPS concentrations at pH 5.2 (at the time point of slightly less than 3 hours of fermentation in the case of without heat treatment, and at the time point of about 4 hours of fermentation in the case of heating at 60°C for 5 minutes), pH 4.6 (at the time point of slightly less than 5 hours of fermentation in the case of without heat treatment, and at the time point of about 6 hours of fermentation in the case of heating at 60°C for 5 minutes), and at the time point of 24 hours of fermentation were improved as compared with the case without heat treatment (Fig. 9).

From this, it was confirmed that an effect of increasing production of EPS begins to appear around 4 hours after the start of fermentation.

After heat treatment at 80°C for 1 minute, a significant decrease in viable cell count was confirmed, but the viable cell count and the EPS concentration at the time point of 24 hours of fermentation were the highest as compared with other cases (Figs. 8 and 9). From these results, it was considered that when bacterial cells of lactic acid bacteria are cultured after being treated under a condition that a lag phase is prolonged by 10% or more, the effect of increasing production of EPS begins to appear after a middle phase of logarithmic growth. More specifically, the middle phase of logarithmic growth was considered to be after the time point when pH of the fermentation solution reached 5.2 or after the time point of about 2.5 hours of fermentation. It was considered that the effect of increasing production of EPS begins to appear more significantly between a late phase of logarithmic growth and the time point of 24 hours. More specifically, the late phase of logarithmic growth was considered to be after the time point when pH of the fermentation solution reached 4.6 or after the time point of about 4 to 5 hours of fermentation.

[Table 10]

| Inoculation amount | pH | Pretreatment conditions | Culture time | EPS concentration (μg/ml) | | Cell count (cfu/mL) |
|---|---|---|---|---|---|---|
| | | | | ave | SD | |
| 1.00% | pH5.2 | Without treatment | 2:54 | 44.6 | 1.8 | 5.8.E + 07 |
| | | 60°C5min | 4:12 | 46.8 | 0.6 | 5.4.E + 07 |
| | | 80°C1min | *No data because recovery was impossible | | | Sixth power or less |
| | pH4.6 | Without treatment | 4:52 | 62.9 | 0.6 | 5.2.E + 07 |
| | | 60°C5min | 5:59 | 86.2 | 2.6 | 6.3.E + 07 |
| | | 80°C1min | *No data because recovery was impossible | | | Sixth power or less |
| | - | Without treatment | 24 | 83.5 | 1.2 | 1.4.E + 07 |
| | | 60°C5min | 24 | 94.9 | 0.4 | 1.6.E + 07 |
| | | 80°C1min | 24 | 107.0 | 4.8 | 7.9.E + 07 |

[Summary]

As described above, it was found that before culturing various exopolysaccharide-producing bacteria, a concentration of EPS produced in a culture solution increases by treatment for prolonging a lag phase such as heat (high heat), osmotic pressure (high osmotic pressure), pH (high pH), ultrasonic treatment, ultraviolet irradiation treatment, high-pressure treatment, cell wall-degrading enzyme treatment, antibacterial peptide treatment, and freeze-thawing treatment, and there is a positive correlation between a length of the lag phase and the concentration of EPS in the culture solution (see Figs. 1 to 6). Therefore, production of an exopolysaccharide can be promoted by culturing an exopolysaccharide-producing bacterium after treating the exopolysaccharide-producing bacterium under a condition that a lag phase is prolonged.

As described above, there can be provided a method for producing an exopolysaccharide, the method including a step of: treating various exopolysaccharide-producing bacteria under a condition that a lag phase is prolonged, particularly a lag phase is prolonged by 10% or more; and producing an exopolysaccharide by culturing the treated exopolysaccharide-producing bacterium in a medium.

Industrial Applicability

The present invention provides a method for producing an exopolysaccharide derived from an exopolysaccharide-producing bacterium, and a method for producing a food containing an exopolysaccharide, and prevents or improves a disease or a condition that can be improved by a function of the exopolysaccharide, and supports maintaining and improving health of non-diseased people. According to the present invention, it is possible to provide a food composition that maintains and improves health of people and a method for producing a food. In addition, the present invention can realize improvement in nutrition, ensure a healthy life, and promote welfare of various people.

## Claims

1. A method for producing an exopolysaccharide, the method comprising:

   treating an exopolysaccharide-producing bacterium under a condition that a lag phase is prolonged by 10% or more; and
   producing an exopolysaccharide by culturing the treated exopolysaccharide-producing bacterium in a medium.

2. The production method according to claim 1, wherein the producing an exopolysaccharide is performed for 4 to 72 hours.

3. The production method according to claim 1, wherein the producing an exopolysaccharide comprises a lag phase prolonged by 10%.

4. The production method according to claim 1, comprising at least one of a concentrating an exopolysaccharide and a

purifying an exopolysaccharide.

5. The production method according to claim 1, wherein the exopolysaccharide-producing bacterium is any bacterium selected from a bacterium belonging to the genus *Lactobacillus,* a bacterium belonging to the genus *Lactococcus,* a bacterium belonging to the genus *Streptococcus,* and a bacterium belonging to the genus *Bifidobacterium.*

6. The production method according to claim 1, wherein the exopolysaccharide-producing bacterium is any bacterium selected from a bacterium belonging to *Lactobacillus delbrueckii,* a bacterium belonging to *Lactococcus lactis,* a bacterium belonging to *Streptococcus thermophilus,* and a bacterium belonging to *Bifidobacterium breve.*

7. The production method according to claim 1, wherein the *Lactobacillus delbrueckii* is a bacterium belonging to *Lactobacillus delbrueckii* ssp. *bulgaricus.*

8. A composition comprising an exopolysaccharide-producing bacterium of any of a bacterium belonging to the genus *Lactobacillus,* a bacterium belonging to the genus *Lactococcus,* a bacterium belonging to the genus *Streptococcus,* and a bacterium belonging to the genus *Bifidobacterium,* treated under a condition that a lag phase is prolonged by 10% or more, for use of an exopolysaccharide as a functional ingredient.

9. The composition according to claim 8, wherein the exopolysaccharide-producing bacterium is any of a bacterium belonging to *Lactobacillus delbrueckii,* a bacterium belonging to *Lactococcus lactis,* a bacterium belonging to *Streptococcus thermophilus,* and a bacterium belonging to *Bifidobacterium breve.*

10. A bacterium belonging to *Lactobacillus delbrueckii* having a lag phase of 152 minutes or more, a bacterium belonging to *Lactococcus lactis* having a lag phase of 236 minutes or more, a bacterium belonging to *Streptococcus thermophilus* having a lag phase of 199 minutes or more, and a bacterium belonging to *Bifidobacterium breve* having a lag phase of 425 minutes or more.

11. A composition comprising any of a bacterium belonging to *Lactobacillus delbrueckii* having an ability to produce an exopolysaccharide of 30 mg/kg or more, a bacterium belonging to *Lactococcus lactis* having an ability to produce an exopolysaccharide of 2.2 mg/kg or more, a bacterium belonging to *Streptococcus thermophilus* having an ability to produce an exopolysaccharide of 69 mg/kg or more, and a bacterium belonging to *Bifidobacterium breve* having an ability to produce an exopolysaccharide of 4.2 mg/kg or more.

12. A culture of *Lactobacillus delbrueckii,* the culture comprising 300 mg/kg or more of an exopolysaccharide produced by *Lactobacillus delbrueckii,* and a composition comprising the culture.

13. A culture of a bacterium belonging to *Lactococcus lactis,* the culture comprising 2.2 mg/kg or more of an exopolysaccharide produced by *Lactococcus lactis,* and a composition comprising the culture.

14. A culture of *Streptococcus thermophilus,* the culture comprising 69 mg/kg or more of an exopolysaccharide produced by *Streptococcus thermophilus,* and a composition comprising the culture.

15. A culture of *Bifidobacterium breve,* the culture comprising 4.2 mg/kg or more of an exopolysaccharide produced by *Bifidobacterium breve,* and a composition comprising the culture.

16. A method for producing a fermented milk, the method comprising fermenting a raw material milk with one or two or more lactic acid-producing bacteria including exopolysaccharide-producing bacteria treated so that a lag phase is prolonged by 10% or more to obtain a fermented milk containing an exopolysaccharide.

17. The production method according to claim 16, wherein the producing an exopolysaccharide is performed for 4 to 72 hours.

18. The production method according to claim 16, wherein the producing an exopolysaccharide comprises a lag phase prolonged by 10%.

19. The production method according to claim 16, wherein the exopolysaccharide-producing bacteria treated so that a lag phase is prolonged by 10% or more comprise a bacterium belonging to *L. delbrueckii* ssp. *bulgaricus,* and a content of the exopolysaccharide in the fermented milk after the fermenting is 30 mg/kg or more;

the exopolysaccharide-producing bacteria treated so that a lag phase is prolonged by 10% or more comprise a bacterium belonging to *Lactococcus lactis,* and a content of the exopolysaccharide in the fermented milk after the fermenting is 2.2 mg/kg or more;
the exopolysaccharide-producing bacteria treated so that a lag phase is prolonged by 10% or more comprise a bacterium belonging to *Streptococcus thermophilus,* and a content of the exopolysaccharide in the fermented milk after the fermenting is 69 mg/kg or more; or
the exopolysaccharide-producing bacteria treated so that a lag phase is prolonged by 10% or more comprise a bacterium belonging to *Bifidobacterium breve,* and a content of the exopolysaccharide in the fermented milk after the fermenting is 4.2 mg/kg or more.

**20.** The production method according to claim 16, wherein the lactic acid-producing bacteria further comprise a bacterium belonging to *Streptococcus thermophilus.*

**21.** The production method according to any one of claims 16 to 20, wherein the lactic acid-producing bacteria further comprise a bacterium belonging to *L. bulgaricus.*

**22.** A method for improving an ability to produce an exopolysaccharide of an exopolysaccharide-producing bacterium, the method comprising treating the exopolysaccharide-producing bacterium under a condition that a lag phase is prolonged.

**23.** The production method according to claim 1 or 16, or the method according to claim 22, wherein the treatment under a condition that a lag phase is prolonged comprises any treatment selected from the group consisting of heat treatment, osmotic pressure treatment, and pH treatment.

# FIG. 1

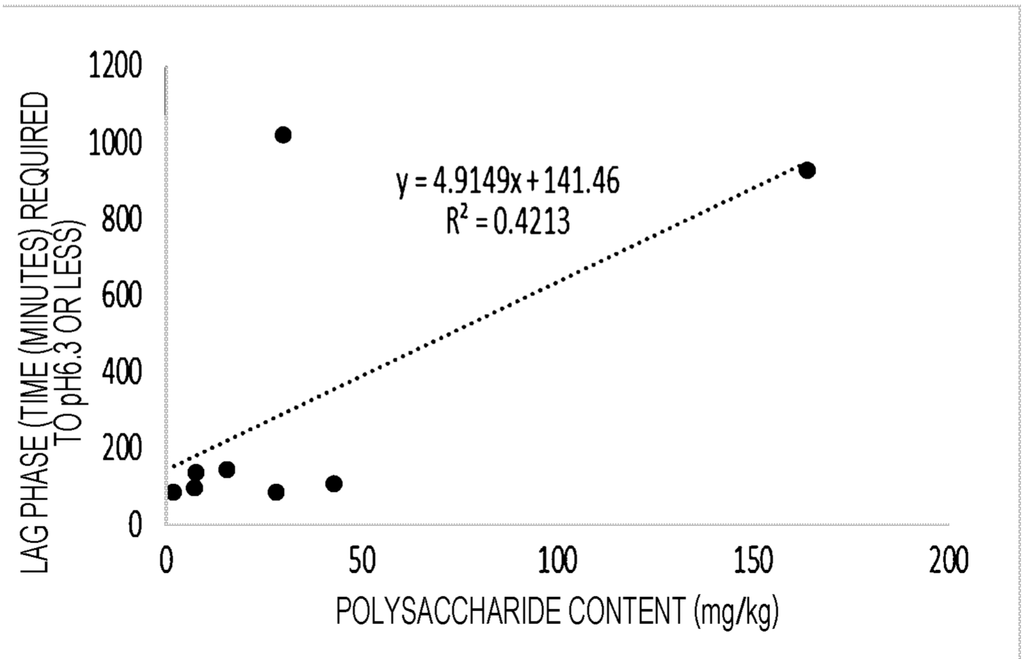

1200
1000
800
600
400
200
0
0
50
100
150
200
y = 4.9149x + 141.46
$R^2$ = 0.4213
LAG PHASE (TIME (MINUTES) REQUIRED TO pH6.3 OR LESS)
POLYSACCHARIDE CONTENT (mg/kg)


# FIG. 2

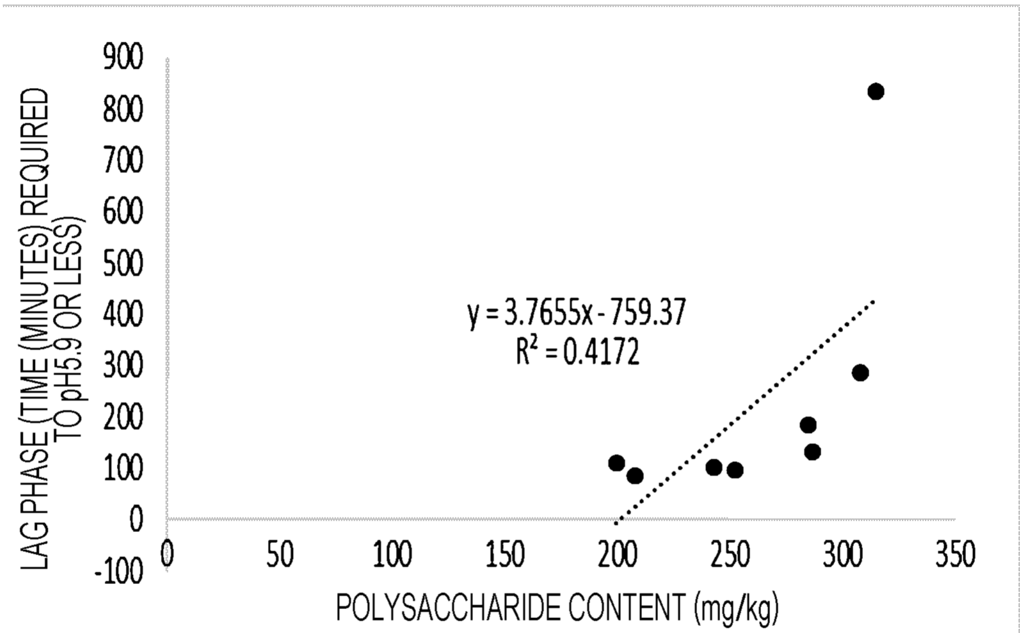

900
800
700
600
500
400
300
200
100
0
-100
0
50
100
150
200
250
300
350
y = 3.7655x - 759.37
$R^2$ = 0.4172
LAG PHASE (TIME (MINUTES) REQUIRED TO pH5.9 OR LESS)
POLYSACCHARIDE CONTENT (mg/kg)

FIG. 3

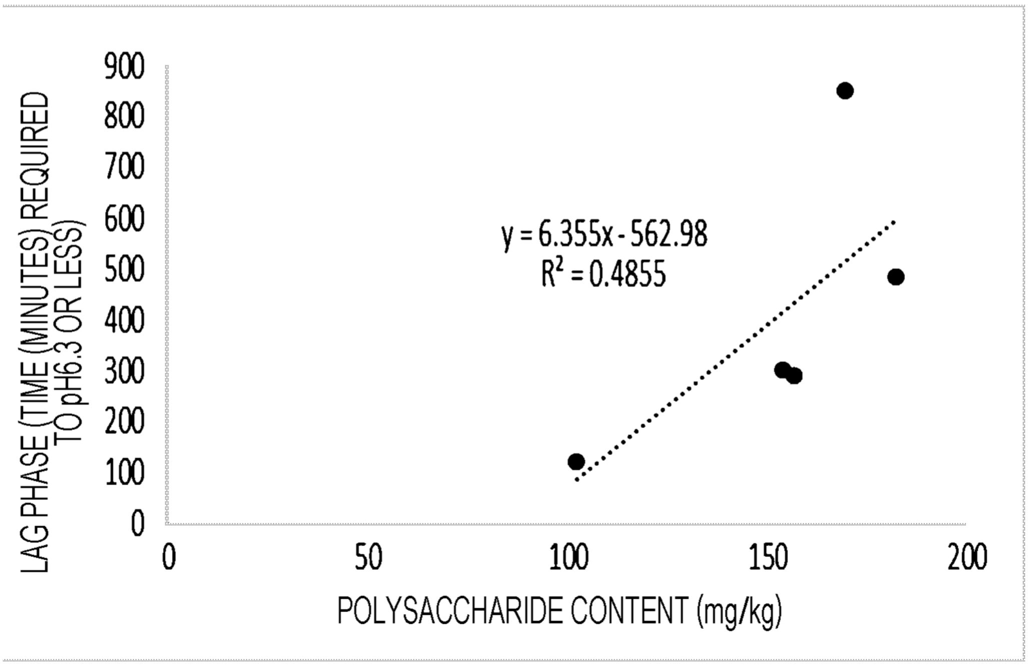
900
800
700
600
500
400
300
200
100
0
0
50
100
150
200
LAG PHASE (TIME (MINUTES) REQUIRED TO pH6.3 OR LESS)
POLYSACCHARIDE CONTENT (mg/kg)
y = 6.355x - 562.98
R² = 0.4855

FIG. 4

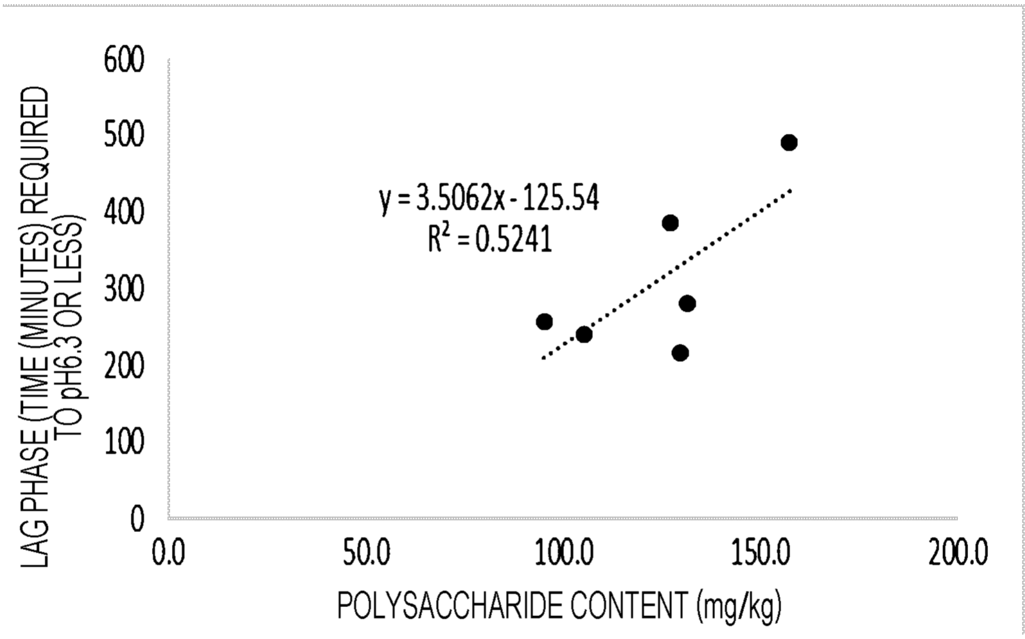
600
500
400
300
200
100
0
0.0
50.0
100.0
150.0
200.0
LAG PHASE (TIME (MINUTES) REQUIRED TO pH6.3 OR LESS)
POLYSACCHARIDE CONTENT (mg/kg)
y = 3.5062x - 125.54
R² = 0.5241

FIG. 5

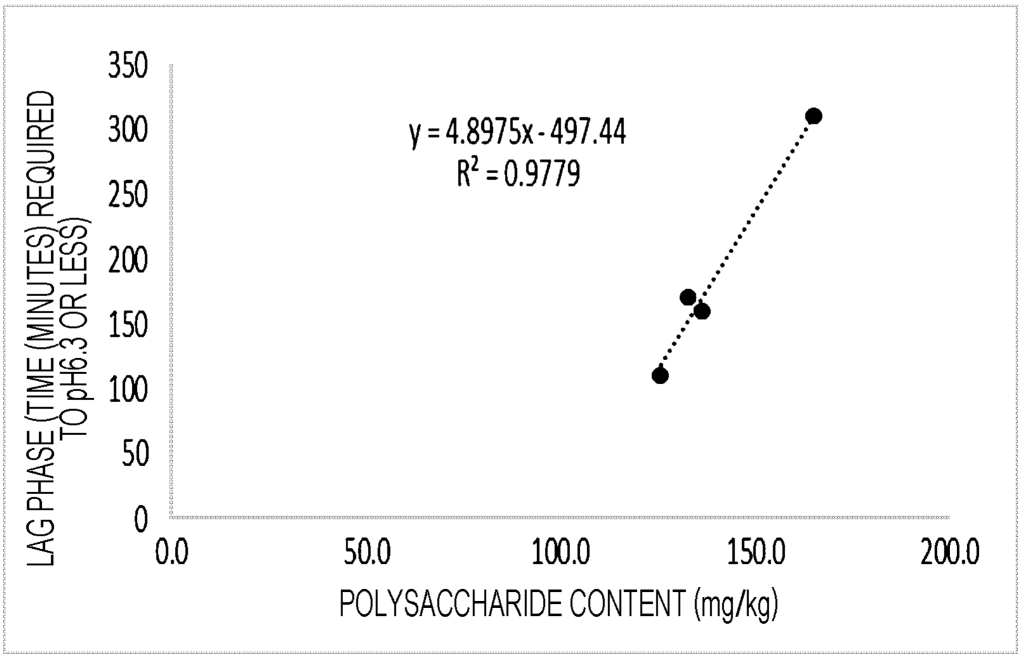

LAG PHASE (TIME (MINUTES) REQUIRED TO pH6.3 OR LESS)
350
300
250
200
150
100
50
0
y = 4.8975x - 497.44
$R^2$ = 0.9779
0.0
50.0
100.0
150.0
200.0
POLYSACCHARIDE CONTENT (mg/kg)


FIG. 6

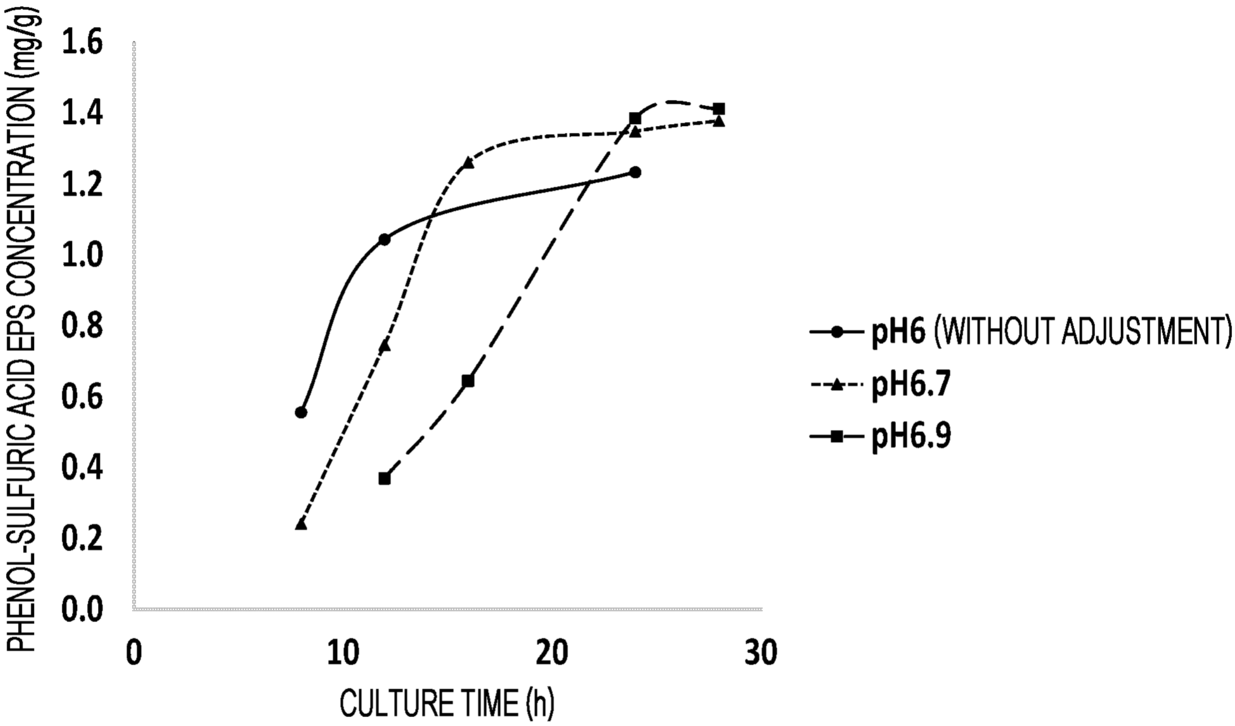

PHENOL-SULFURIC ACID EPS CONCENTRATION (mg/g)
1.6
1.4
1.2
1.0
0.8
0.6
0.4
0.2
0.0
0
10
20
30
CULTURE TIME (h)
pH6 (WITHOUT ADJUSTMENT)
pH6.7
pH6.9

FIG. 7

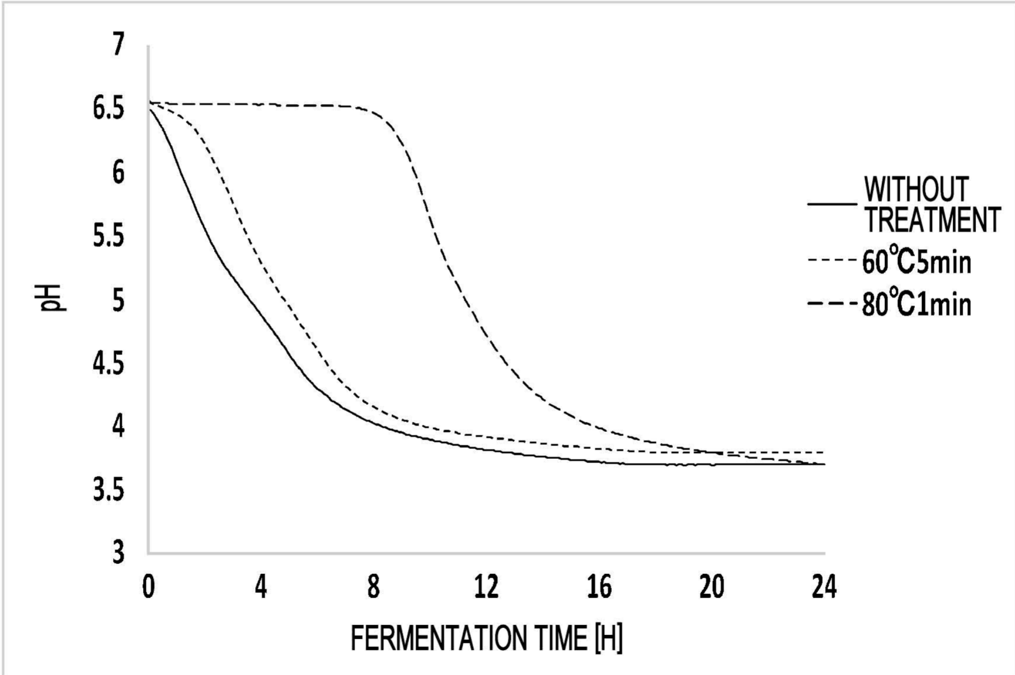
7
6.5
6
5.5
5
4.5
4
3.5
3
pH
0
4
8
12
16
20
24
FERMENTATION TIME [H]
WITHOUT TREATMENT
60°C5min
80°C1min

FIG. 8

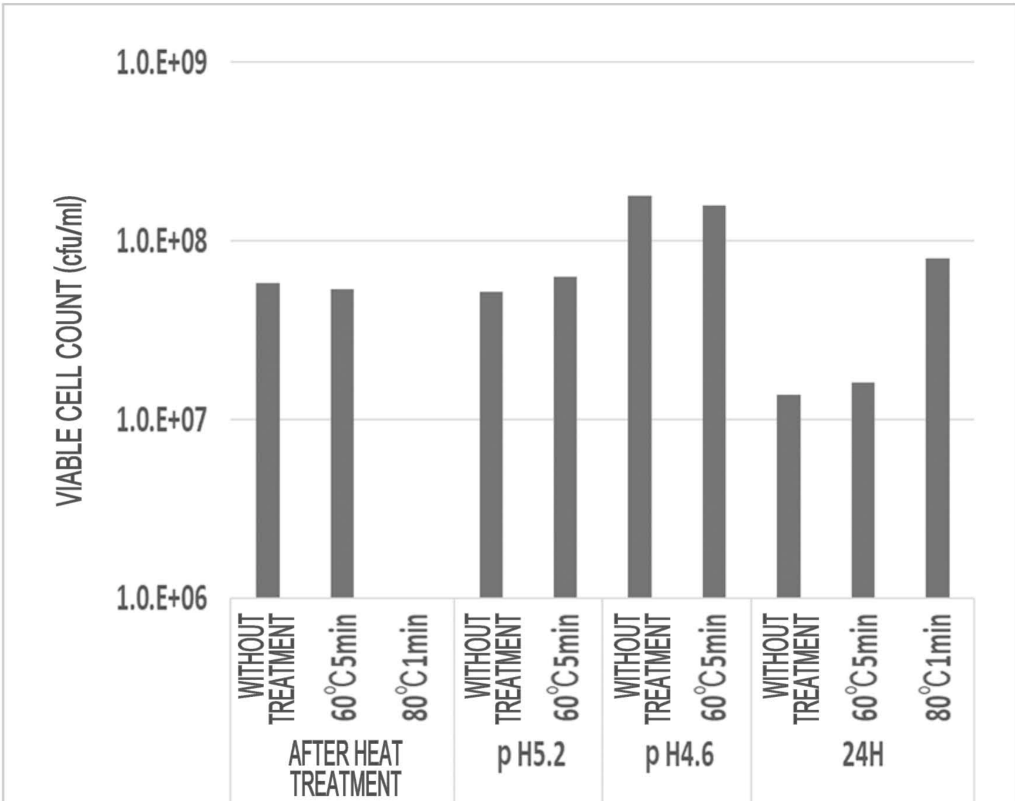
1.0.E+09
1.0.E+08
1.0.E+07
1.0.E+06
VIABLE CELL COUNT (cfu/ml)
WITHOUT TREATMENT
60°C5min
80°C1min
AFTER HEAT TREATMENT
WITHOUT TREATMENT
60°C5min
p H5.2
WITHOUT TREATMENT
60°C5min
p H4.6
WITHOUT TREATMENT
60°C5min
80°C1min
24H

*FIG. 9*

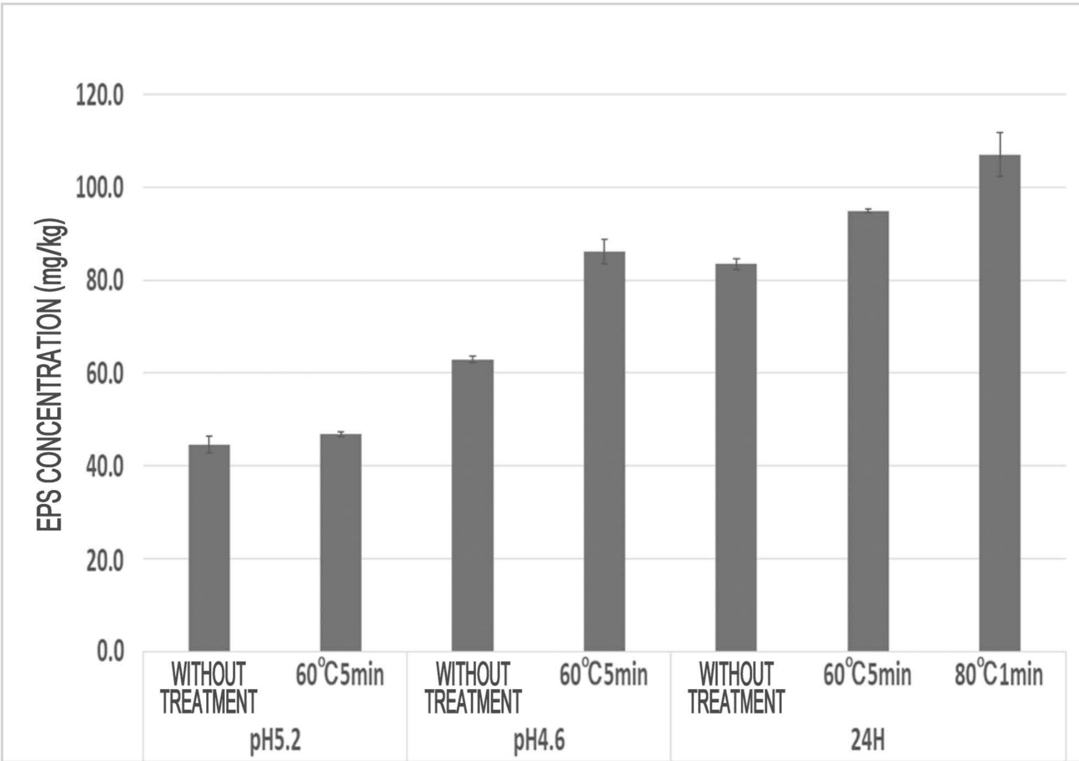
EPS CONCENTRATION (mg/kg)
120.0
100.0
80.0
60.0
40.0
20.0
0.0
WITHOUT TREATMENT
60°C5min
WITHOUT TREATMENT
60°C5min
WITHOUT TREATMENT
60°C5min
80°C1min
pH5.2
pH4.6
24H

## INTERNATIONAL SEARCH REPORT

International application No. **PCT/JP2024/020804**

### A. CLASSIFICATION OF SUBJECT MATTER

***C12P 19/04***(2006.01)i; ***A23C 9/123***(2006.01)i; ***A23L 29/269***(2016.01)i; ***C12N 1/20***(2006.01)i
FI: C12P19/04 C; C12N1/20 A; A23C9/123; A23L29/269

According to International Patent Classification (IPC) or to both national classification and IPC

### B. FIELDS SEARCHED

Minimum documentation searched (classification system followed by classification symbols)

C12P19/00-19/64; C12N1/20; A23C9/00-9/20; A23L29/269

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | WO 2021/132418 A1 (MEIJI CO., LTD.) 01 July 2021 (2021-07-01)<br>test example 9, fig. 1, claim 8 | 1-12, 16-19, 21-23 |
| X | JP 2014-027925 A (MEIJI CO., LTD.) 13 February 2014 (2014-02-13)<br>paragraphs [0013], [0017], [0023] | 1-11, 13, 16-19, 21-23 |
| X | NGUYEN, PT., et al. , Exopolysaccharide production by lactic acid bacteria: the manipulation of environmental stresses for industrial applications. AIMS MICROBIOLOGY. 17 November 2020, vol. 6, no. 4, pp. 451-469<br>p. 452, "1. Introduction," first, second paragraphs, p. 454, "3. The role of EPSs for LAB stress resistance," second paragraph, p. 458, first paragraph, p. 458, last paragraph to p. 459, first paragraph, p. 459, "5.3. Co-cultivation," first paragraph, p. 460, first paragraph, fig. 3, 5 | 1-12, 14, 16-23 |
| X | PRASANNA, P. H. P., et al., Screening human intestinal Bifidobacterium strains for growth, acidification, EPS production and viscosity potential in low-fat milk, INTERNATIONAL DAIRY JOURNAL, 2012, vol. 23, pp. 36-44<br>table 2, p. 37, "2.2. Preparation of fermented milk" | 1-6, 8-11, 15-19, 22 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 August 2024** | **20 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/020804**

**C. DOCUMENTS CONSIDERED TO BE RELEVANT**

| Category* | Citation of document, with indication, where appropriate, of the relevant passages | Relevant to claim No. |
|---|---|---|
| X | JP 2020-115783 A (MEIJI CO., LTD.) 06 August 2020 (2020-08-06)<br>claim 1, paragraph [0028] | 8-12, 14 |
| X | DE VUYST, L., et al., Production by and isolation of exopolysaccharides from Streptococcus thermophilus grown in a milk medium and evidence for their growth-associated biosynthesis, JOURNAL OF APPLIED MICROBIOLOGY, 1998, vol. 84, pp. 1059-1068<br>particularly tables 2-5 | 8-11, 14 |

Form PCT/ISA/210 (second sheet) (July 2022)

**INTERNATIONAL SEARCH REPORT**
**Information on patent family members**

International application No.
**PCT/JP2024/020804**

| Patent document cited in search report | Publication date (day/month/year) | Patent family member(s) | Publication date (day/month/year) |
|---|---|---|---|
| WO 2021/132418 A1 | 01 July 2021 | EP 4083187 A1<br>test example 9, fig. 1, claim 8<br>US 2023/0052298 A1<br>CN 115135748 A | |
| JP 2014-027925 A | 13 February 2014 | (Family: none) | |
| JP 2020-115783 A | 06 August 2020 | CN 111466439 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005194259 A
- JP 5177728 B
- WO 2011065300 A
- JP 5971949 B
- JP 2014027925 A
- JP 6209371 B
- WO 2014084340 A
- JP 6392668 B
- JP 2019062782 A
- JP 7109895 B
- JP 2022103317 A
- JP 20172169276 A
- JP 6621065 B
- JP 2020156341 A
- JP 2022045812 A
- JP 2010104376 A
- JP 4759643 B
- JP 2000247895 A
- WO 2022220154 A


### Non-patent literature cited in the description


- **JURASKOVA et al.** Exopolysaccharides Produced by Lactic Acid Bacteria:From Biosynthesis to Health-Promoting Properties.. *Foods.*, 2022, vol. 11, 156
- **PHU-THO NGUYEN et al.** Exopolysaccharide production by lactic acid bacteria:the manipulation of environmental stresses for industrial applications.. *AIMS Microbiology*, 2020, vol. 6 (4), 451-469
- **HUU THANH NGUYEN et al.** Stochastic exposure to sub-lethal high temperature enhances exopolysaccharides (EPS) excretion and improves Bifidobacterium bifidum cell survival to freeze-drying.. *Biochemical Engineering Journal*, 2014, vol. 88, 85-94
- **ROBERT L. BERTRAND.** Lag Phase Is a Dynamic, Organized, Adaptive, and Evolvable Period That Prepares Bacteria for Cell Division.. *Journal of Bacteriology*, 2019, vol. 201 (7), e00697-18
- **L. M. VALIK et al.** Characterization of the growth of Lactobacillus rhamnosus GG in milk at suboptimal temperatures.. *Journal of Food and Nutrition Research*, 2008, vol. 47 (2), 60-67
- A taxonomic note on the genus Lactobacillus:Description of 23 novel genera, emended description of the genus Lactobacillus Beijerinck 1901, and union of Lactobacillaceae and Leuconostocaceae. *INTERNATIONAL JOURNAL OF SYSTEMATIC AND EVOLUTIONARY MICROBIOLOGY*, 15 April 2020, vol. 70 (4)
- **ZIADI et al.** *BioMed Research International*, 2018, vol. 2018